# EUROPEAN PATENT APPLICATION

(11) **EP 1 319 950 A1**
(43) Date of publication of application: **18.06.2003**
(21) Application number: 02258336.3
(22) Date of filing: 03.12.2002
(51) Int. Cl.: G01N 33/50, G01N 33/543, C12Q 1/68, B01L 3/00, B01J 19/00

(54) **Matrix screening method**

(30) Priority: 03.12.2001 US 8571
(71) Applicant: Domantis Limited, Cambridge, CB1 6GS (GB)
(72) Inventor: Tomlinson, Ian, London, W1N 4AL (GB); Holt, Lucy j., London, W1N 4AL (GB)
(74) Representative: Maschio, Antonio

(57) **Abstract**

The invention concerns a method which can be used to screen two or more repertoires of molecules against one another and/or to create combinatorial repertoires by combining two or more repertoires. In particular, the invention relates to a method whereby two repertoires of molecules can be screened such that all members of the first repertoire are tested against all members of the second repertoire for functional interactions. Furthermore, the invention relates to the creation and screening of antibody repertoires by combining a repertoire of heavy chains with a repertoire of light chains such that antibodies formed by the all combinations of heavy and light chains can be screened against one or more target ligands.

## Description

### Field of Invention

The present invention relates to a method which can be used to screen two or more repertoires of molecules against one another and/or to create and screen combinatorial repertoires by combining two or more repertoires. In particular, the invention relates to a method whereby two repertoires of molecules can be screened such that all members of the first repertoire are tested against all members of the second repertoire for functional interactions. Furthermore, the invention relates to the creation and screening of antibody repertoires by combining a repertoire of heavy chains with a repertoire of light chains such that antibodies formed by the all combinations of heavy and light chains can be screened against one or more target ligands.

### Introduction

The mapping and sequencing of different genomes will eventually lead to the cloning of all the proteins expressed by these organisms. In order to create interaction maps of these proteins, two-dimensional screens need to be performed so that the binding of every protein to every other protein can be tested.

Two dimensional screens are also required for a number of other applications. For example, techniques such as mouse immunisation coupled with the production of monoclonal antibodies and *in vitro* selection methods such as phage display have been used to simultaneously generate many different antibodies against many different targets. In order to determine which antibodies bind to which targets these pools need to be deconvoluted, which requires a complex screening procedure.

Furthermore, if small molecule drugs are to be generated against human targets for therapy it would be helpful to determine not only the extent of binding of a given human protein to a putative drug candidate but also the extent (if any) of cross-reaction of the same drug candidate with other human proteins or whether other related drugs are better binders and/or less cross-reactive.

All of these examples call for a technique whereby interactions between members of a first set (or repertoire) of molecules can be rapidly tested against all members of a second set (or repertoire) of molecules. To date, such screens are generally performed by dispensing combinations of reagents into compartmentalised wells or on top of one another in the form of spots on a membrane such that all combinations of reagents to be tested are present in separate wells/spots. Therefore if a repertoire of 100 molecules were to be tested against a different repertoire also consisting of 100 molecules, 10,000 wells/spots would be required to exhaustively cover all combinations of members of the two repertoires. The creation of such discontinuously arranged combinations would require, for a two component interaction, twice as many dispensing 'events' as there are wells or spots, in this case 20,000, in addition to any dispensing events that might be required to facilitate or detect the interactions. As the number of members in each repertoire increases linearly, the number of combinations, and hence dispensing events, increases exponentially. Indeed for a three component interaction, involving, say, a repertoire of only 100 antibody heavy chains, a repertoire of only 100 antibody light chains and a repertoire of only 100 potential antigens, a million 'dispensing' events would be required.

Thus the present invention provides significant advantages over current screening methods available to those of skill in the art. One such method is described in U.S. Pat. No. 6,087,093, in which hybridization probes specific for mutations in the reverse transcriptase gene are placed on membrane strips which are hybridized to a patient sample. The '093 patent does not teach generating two or more repertoires of members wherein each member of each repertoire is arrayed in a series of multiple intersecting lines such that each member of each repertoire is juxtaposed to each other member of each repertoire. The present invention, in addition to being distinct from the methods taught in the '093 patent, has advantages over the '093 methods, in that, the present invention would permit one of skill in the art to screen a repertoire comprising a plurality of reverse transcriptase mutants against a repertoire plurality of patient samples, using fewer dispensing steps than required by following the teachings of the '093 patent.

Other studies have utilized overlapping lines on a solid surface, however, these methods have been applied to sequence analysis, and do not provide teaching of detection of interactions between members of two or more repertoires as is provided by the present invention. PCT publication WO89/10977 teaches a method of determining a polynucleotide sequence by generating on a surface every combination of nucleotides which yield a sequence of a given length. The '977 publication teaches making the oligos by a method that comprises depositing a series of four rows and intersecting columns on a surface, each containing one of the four nucleic acid bases, thus generating the 16 possible dinucleotide combinations. This is repeated, overlaying narrower and narrower rows and columns over the preceeding row or column until the desired sequence length is reached. The surface is then hybridized to a target sample. The '977 publication does not teach detecting interactions between the members of the rows and columns, at the intersection of the rows and columns as is claimed in the present invention. Thus, the present invention is clearly distinct from the teachings of the '977 publication.

### Summary of the Invention

The invention is based on a methodology, called Matrix Screening, which is useful to study all possible interactions between all the members in two or more repertoires of molecules. The method precludes the need to compartmentalise individual combinations of members of these repertoires.

According to a first aspect of the present invention, there is provided a method for screening a first repertoire of molecules against a second repertoire of molecules to identify those members of the first repertoire which interact with members of the second repertoire, comprising :
(a) providing an array of members of the first repertoire juxtaposed with members of the second repertoire which permits interaction of the first repertoire members with the second repertoire members, the array comprising a solid surface, the first repertoire present on a solid surface in a first series of continuous, non-intersecting lines such that each line of the series comprises a member of the first repertoire, and the second repertoire present on a solid surface in a second series of continuous, non-intersecting lines such that each line of the series comprises a member of the second repertoire, such that members of the first repertoire are juxtaposed to members of the second repertoire; and
(b) detecting an interaction between members of the first and second repertoires, thereby identifying a member of the first repertoire that interacts with a member of the second repertoire.

The invention, in its broadest form, provides a method for screening two repertoires of molecules against one another. Individual members of the two repertoires are spatially configured to enable the juxtaposition of all combinations of members of both repertoires. It will be understood that reference herein to "all combinations" (or "all members") does not exclude that certain juxtapositions may not occur, either by chance or by design. However, the invention does require that two repertoires of molecules be screened against each other simultaneously, and excludes the screening of a single repertoire with individual member(s) of a second repertoire. Preferably, "all" refers to at least 50%, 55%, 60%, 65%, 70%, 75%, 80%, 85%, 90%, 95%, 96%, 97%, 98%, 99% or 100% of the members of a repertoire.

According to the invention, juxtaposition can be arrived at by, for example, creating a series of lines for each of the two repertoires, which intersect one another. The lines can be straight, substantially parallel lines, or curves, or combinations thereof; the only restriction is that all members of the first repertoire should be juxtaposed to all members of the second repertoire. Examples of complementary configurations include straight parallel lines, disposed at an angle to straight parallel lines; concentric circles or polygons, used together with a star of radial lines. The skilled person will be able to imagine many other systems being used to achieve a similar spatial configuration of the repertoire members according to the invention, all being characterised by the dispensation of some form of continuous line, stream, channel or flow corresponding to each member of the first repertoire, all of which has the ability to intersect all lines, streams, channels or flows corresponding to all members of the second repertoire. These include tubes for each member of the first repertoire which intersect tubes of the second repertoire, or channels cut in a solid material down which individual repertoire members can flow.

Therefore, according to a second aspect of the invention, there is provided a method wherein members of the both the first repertoire and the second repertoire are arranged in a series of lines, channels or tubes, each containing a member of the first or second repertoires such that the lines, channels or tubes corresponding to the first repertoire and those corresponding to the second repertoire are contacted with one another so that all members of the first repertoire are juxtaposed with all members of the second repertoire.

In the context of the present invention, "a" member can mean one single member or at least one member. Advantageously, it refers to one single member. However, in an alternative aspect the invention also provides the use of groups consisting of more than one member of the repertoire in each line, channel or tube. Preferably, such groups consist of 10 of fewer members, advantageously 5 or fewer, but at least 2.

The advantage of using intersecting lines, channels, streams or flows according to the present invention compared to compartmentalised combinatorial screening in the prior art is that as the size of the individual repertoires grow linearly, so does the number of dispensing steps required to screen all combinations of repertoire members. Thus, whereas screening techniques using wells would require 10,000 dispensing steps to screen a 100 by 100 repertoire, screening according to the present invention requires only 200 dispensing steps. Furthermore, since a single dispensing event is used to spatially array each member of each repertoire, comparison of interactions between individual members of the first repertoire with the members of the second repertoire with which it is juxtaposed will be more accurate. In addition, since the present invention uses intersecting lines rather than spots or intersecting channels rather than wells, less positional accuracy is necessary to ensure that all combinations of possible interactions are tested. Thus, if a two-dimensional screen is performed, and one line corresponding to a member of the first repertoire is offset by, for example, 1mm, since it is arranged at an angle to all the lines from the second repertoire, it will still intersect all of them and therefore all combinations of interactions will still have been successfully tested. If, on the other hand, the spots corresponding to a member of the first repertoire are offset by, for example, 1mm, they may miss the spots corresponding to the members of the second repertoire altogether and therefore many combinations of interactions will not have been tested. Therefore, the present invention is not only well suited to automated methods of screening but also to manual methods, where positional accuracy cannot be guaranteed and the number of dispensing events must be limited.

As described above, the lines, channels or tubes can be arranged in a variety of formats and can be arranged on a single support, or a plurality of supports. In the simplest configuration, molecules can be manually drawn out in the form of lines on a single support, for example on a nitrocellulose membrane. These lines can also be applied to suitable supports using robotic techniques, which allow the accuracy and density of arrays to be increased to great advantage in the present invention. In an advantageous aspect of the invention, a multi-support system can be used, wherein arrays of lines are prepared on separate supports which are then juxtaposed in order to assess interaction between the members of the repertoires.

Accordingly, in a third aspect of the invention a method is provided for screening a first repertoire of molecules against a second repertoire of molecules to identify one or more members of the first repertoire which interact with one or more members of the second repertoire, comprising :
(a) providing an array of members of the first repertoire juxtaposed with members of the second repertoire which permits interaction of the first repertoire members with the second repertoire members, the array comprising the first repertoire present in the lumen of a first series of non-intersecting tubes, such that each tube of the series comprises a member of the first repertoire, and further comprising the second repertoire present in the lumen of a second series of non-intersecting tubes, such that each tube of the series comprises a member of the second repertoire, such that the lumen of each of the first series of tubes intersects with the lumen of each of the second series of tubes, such that members of the first repertoire are juxtaposed to members of the second repertoire.
(b) detecting an interaction between members of the first and second repertoires, thereby identifying a member of the first repertoire that interacts with a member of the second repertoire.

The present invention can also be applied to higher dimensional arrays, for example, those with 3 dimensions. Thus, three component interactions, such as enzyme, substrate and cofactor can be screened using lines, channels or tubes that are arranged in 3 dimensions. Alternatively, the three components could be antibody heavy chain, antibody light chain and antigen, and repertoires thereof can be screened in three dimensions. The screening of repertoires in 2, 3 or higher dimensions according to the present invention is highly advantageous as it reduces the number of dispensing (or pipetting) events that would be required to perform a comprehensive combinatorial screen. Thus, the screening of two repertoires, of, say, 300 members against one another using conventional techniques in the prior art would require at least 90,000 separate dispensing events and the screening of three repertoires, of, say, 300 members against one another would require at least 2.7 million dispensing events. By contrast, the present invention reduces the number of dispensing events to comprehensively screen the same repertoires to 600 or 900, respectively, a huge saving in terms of time and labour.

According to a fourth aspect of the present invention, therefore, there is provided a method for screening first, second and third repertoires of molecules against each other to identify those members of the first, second and third repertoires which interact, comprising :
(a) providing an array of members of first, second, and third repertoires juxtaposed to each other which permits interaction of the first, second and third repertoire members, the array comprising three solid surfaces, a member of the first repertoire present on a first solid surface of the array, a member of the second repertoire present on a second solid surface of the array, and a member of the third repertoire present on a third solid surface of the array, such that each of the first, second, and third solid surfaces of the array intersect, such that members of the first, second and third repertoires are juxtaposed; and
(b) detecting an interaction between members of the first, second and third repertoires, thereby identifying members of the first, second and third repertoires that interact.

The invention provides an alternate method of screening first, second and third repertoires of molecules against each other to identify those members of the first, second and third repertoires which interact, comprising :
(a) providing an array of members of first, second and third repertoires, juxtaposed to each other which permits interaction of the first, second and third repertoire members, the array comprising a solid surface, the first repertoire present on a solid surface in a first series of continuous, non-intersecting lines such that each line of the series comprises a member of the first repertoire, the second repertoire present on a solid surface in a second series of continuous, non-intersecting lines such that each line of the series comprises a member of the second repertoire, and the third repertoire present on a solid surface in a series of continuous, non-intersecting lines such that each line of the series comprises a member of the third repertoire, and such that members of the first, second and third repertoire is juxtaposed to other members of the first, second, and third repertoires; and
(b) detecting an interaction between members of the first, second and third repertoires, thereby identifying members of the first, second and third repertoires that interact.

A multidimensional array can be created in a number of ways. Advantageously, a third dimension is created by stacking filters or other such membranes and relying on capillary action for transferring molecules, or by forcing molecules through the stack by a means such as electrophoresis or osmosis or by piercing the stack or by the use of permeable filters to create the stack.

Moreover, a third dimension can be created by stacking non-permeable layers which at the intersections of channels (for the first and second repertoires) have holes which (once the layers are stacked) form an additional set of channels in a third dimension along which members of a third repertoire can pass.

In a further embodiment, the third dimension can be created using a block of gel or similar such substance, which can be injected with members of the first, second and third repertoires along the x, y and z faces, respectively, thus creating channels in a three-dimensional space which form the array.

Still further, the matrix of interactions between members of the first, second (and optionally third) repertoires of molecules can be created using a network of intersecting tubes or semipermeable tubes laid adjacent to one another.

The members of the first, second (and optionally third) repertoires of molecules can be replaced over time with different members from the same repertoires so that a new combination or set of interactions can be screened.

Since the present invention can be used to rapidly screen multicomponent and multi-chain interactions, it can also be applied to the simultaneous creation and screening of combinatorial libraries of molecules, for example, antibody or T cell receptor libraries. Thus instead of generating a large combinatorial library of antibodies by combining the heavy and light chain genes and then separately screening the resulting pairings, the pairings themselves can be generated according to the invention and, optionally screened against one or more target antigens. Thus, say, 1000 heavy chains could be drawn as lines in one dimension, and a 1000 light chains can be drawn as lines in another, such that all the heavy chain lines intersect all the light chain lines, forming at their intersection fully functional and folded antibody molecules, which can then be screened with a juxtaposed antigen, for example coated on a further support which is brought into contact with the intersecting heavy and light chain lines. According to this embodiment, all combinations of 1000 heavy chains and 1000 light chains will have been screened, i.e. a total of 1 million different antibodies, using only 2000 dispensing events, rather than the 1 million that would have to be used according to screening techniques in the prior art. This provides a rapid way for 'naive' screening for specific interactions. Thus, for example, a repertoire of heavy chains and a repertoires of light chains, the members (or any related member) of which have never been in contact or selected against a given target antigen (or a related target antigen thereof) can be screened against the target antigen to identify a specific binding heavy and light chain pairing.

Thus, in a fifth aspect of the present invention a method is provided for creating and screening a combinatorial library of two-chain polypeptides, each of which comprises one member of a first repertoire and one member of a second repertoire, which method comprises the step of providing an array of members of the first repertoire juxtaposed with the members of the second repertoire which permits interaction of the first repertoire members and the second repertoire members, the array comprising a solid surface wherein the first and second repertoires of single chain polypeptides are present on a solid surface in a first and second series of continuous, non-intersecting lines, respectively, such that each line of the first series intersects with each line of the second series, such that members of the first repertoire are juxtaposed members of the second repertoire, thereby generating two-chain polypeptides at the intersection of the first and second series, thereby creating a combinatorial library of two-chain polypeptides.

Preferably, the combinatorial library is an antibody or T cell receptor library and the two repertoires consist of heavy and light chains (in the case of an antibody library) or alpha and beta chains (in the case of a T cell receptor library).

The combinatorial library so produced is preferably screened for interactions with more than one target molecule. Thus, the target molecule can be provided in the form of a group of target molecules, or a repertoire thereof, and screened in a three-dimensional array as described herein.

Preferably, the method according to the invention can be used such that a three-chain polypeptide library is created (and optionally screened) using first, second and third repertoires of molecules in three dimensions.

The invention provides a method for creating a combinatorial library of three chain polypeptides wherein each member of the library comprises one member of a first repertoire of single chain polypeptides, one member of a second repertoire of single chain polypeptide, and one member of a third repertoire of single chain polypeptides, which method comprises the step of providing an array of members of first, second, and third repertoires juxtaposed to each other which permits interaction of the first, second and third repertoire members, the array comprising three solid surfaces, a member of the first repertoire present on a first solid surface of the array, a member of the second repertoire present on a second solid surface of the array, and a member of the third repertoire present on a third solid surface of the array, such that each of the first, second, and third solid surfaces of the array intersect, such that members of the first, second and third repertoires are juxtaposed, thereby generating three-chain polypeptides at the intersection of the first, second, and third solid surfaces, thereby creating a combinatorial library of three-chain polypeptides.

The invention also provides an alternate method for creating a combinatorial library of three-chain polypeptides, wherein each member of the library comprises one member of a first repertoire of single chain polypeptides, one member of a second repertoire of single chain polypeptides, and one member of a third repertoire of single chain polypeptides, which method comprises the step of providing an array, comprising a solid surface, wherein the first, second, and third repertoires of single chain polypeptides are present on a solid surface in a first, second, and third series of continuous, non-intersecting lines, respectively, such that each line of the first series intersects with each line of the second and third series, each line of the second series intersects with each line of the first and third series, and each line of the third series intersects with the first and second series, such that members of the first, second and third repertoires are juxtaposed to each other, thereby generating three-chain polypeptides at the intersection of the first, second, and third series, thereby creating a combinatorial library of three-chain polypeptides.

The pattern of interactions between the first, second (and optionally third) repertoires can be used to identify positive interactions, negative interactions, specific interactions or cross-reactive interactions, or to construct a phylogenic tree inferring the similarity between members of the first repertoire (using the pattern of interactions with the second and/or, optionally third, repertoires), of the second repertoire (using the pattern of interactions with the first and/or, optionally third, repertoires) and/or of the third repertoire (using the pattern of interactions with members the first and/or second repertoires).

In one embodiment, the interactions between the first and second (and optionally third) repertoires can be used to construct a phylogenetic tree by following the assumption that shared characteristics (in this case binding characteristics) are likely to indicate shared ancestry. If, for example, a collection of serum albumins from different animals was purified and antibodies were raised against a mixture of the albumins and then each antibody was separately probed for binding to each albumin (using the matrix approach of the present invention) then it might be possible to group the albumins according to their shared binding characteristics. There are several phylogenetic approaches known in the art, which have previously been applied to DNA data or to macro characteristics, (presence or absence of certain fenestrations in the skull, type of jaw hinge, etc). Such methods of constructing phylogenetic analysis of, for example evolved binding characteristics are known to those of skill in the art, and may be found, for example, in Evolution (Ridley, M, 1996, 2^{nd} Ed. Blackwell Scientific).

Since many of the interactions that will be screened according to the present invention involve polypeptides that have been derived, directly or indirectly, by expression of nucleic acid sequences, it is highly advantageous that the nucleic acids themselves are arranged in lines, channels or tubes according to the invention and expressed to produce their corresponding polypeptides. In this way, intersecting polypeptides from each of the two repertoires will be expressed together. This can assist their association, particularly when the association of the two repertoire members depends on co-operative folding, for example, as in the case of antibodies. In addition, information regarding the interactions of members of the repertoires will be spatially linked to the genetic information which encodes them. This genetic information can be determined by calculating the co-ordinates of the interaction and isolating the corresponding nucleotide sequence data from any point on its line, channel or tube or by isolating the nucleotide sequence data from the intersection itself.

Accordingly, in a sixth aspect of the present invention, a method is provided whereby one or more of the first, second and, optionally, third repertoires comprise a plurality of nucleic acid molecules which are expressed to produce their corresponding polypeptides *in situ* in the array.

Since the present invention concerns the rapid and efficient screening of two or more repertoires against one another, any currently employed techniques for enhancing or disrupting molecular interactions can be used with the invention. Thus, one repertoire can consist of variants of a free hapten and the other repertoire can consist of selected anti-hapten antibodies. By arranging both repertoires in close proximity to an immobilised version of the target hapten molecule the screen can be used to identify those antibodies that are competed for binding to the immobilised target hapten by binding to certain free hapten variants. In this case, the lack of binding would be considered a positive result. Controls for such an experiment can include a line of water alongside the free haptens and a line of non-hapten binding antibodies alongside the anti-hapten antibodies. Alternatively, a single free hapten could be used to disrupt binding of members of a repertoire of anti-hapten antibodies to members of a repertoire of different immobilised hapten variants. Other third molecules might include substances that enhance binding of the repertoire members to one another, which can be used itself in the form of a repertoire according to the invention. In this way, a target molecule could be immobilised on a solid support and intersecting repertoires of binders and binder enhancers could be brought into contact with the target molecule. Those skilled in the art will envisage many different combinations of such molecules and repertoire members.

Accordingly, in a seventh aspect of the present invention a method is provided for screening a first repertoire of molecules against a second repertoire of molecules to identify members of the first and second repertoires whose interactions with one another are dependant on the presence or absence of a third molecule or set of molecules, comprising:
(a) providing an array of members of the first repertoire juxtaposed with members of the second repertoire which permits interaction of the first repertoire members with the second repertoire members, the array comprising a solid surface, wherein the first repertoire is present on a solid surface in a first series of continuous, non-intersecting lines such that each line of the series comprises a member of the first repertoire, and the second repertoire is present on a solid surface in a second series of continuous, non-intersecting lines such that each line of the series comprises a member of the second repertoire, such that members of the first repertoire are juxtaposed to members of the second repertoire; and
(b) contacting the first and second repertoires with the one or more third molecules;
(c) detecting interactions between members of the first repertoire and members of the second repertoire in the presence of the one or more third molecules, such that members of the first and second repertoires whose interactions with one another are dependent on the presence or absence of the one or more third molecules are identified.

In a further aspect, the present invention provides a method for determining conditions for a biological interaction, which method comprises arranging two or more repertoires of variable parameters such as, but not limited to pH, concentration, temperature, viscosity, ionic strength, buffer composition, a substrate concentration, the presence of denaturants, the presence of renaturants, and the like, in two or more different sets of intersecting lines, channels, or tubes, thus creating all combinations of the two or more different sets of variable parameters at the intersections of the two or more different sets of intersecting lines, channels or tubes, and assaying the biological interaction, wherein the biological interaction is blocked, decreased, enhanced, or not changed, thus indicating which combination of variable conditions is optimal for the biological interaction, thereby determining the conditions for the biological interaction.

In a further aspect, the present invention relates to a method for screening a first and a second repertoire of enzymes to identify those members of the first repertoire and those members of the second repertoire which together participate in a two or more step enzymatic reaction that creates a given product from a given substrate, which method comprises:
(a) providing an array of members of the first repertoire juxtaposed with members of the second repertoire which permits interaction of the first repertoire members and the second repertoire members, the array comprising a solid surface, the first repertoire present on a solid surface in a first series of continuous, non-intersecting lines such that each line of the series comprises a member of the first repertoire, and the second repertoire present on a solid surface in a second series of continuous, non-intersecting lines such that each line of the series comprises a member of the second repertoire, such that members of the first repertoire are juxtaposed to members of the second repertoire;
(b) contacting the array with the substrate; and
(c) detecting the formation of the product at the intersections of the members of the first and second repertoires, thereby identifying members of the first and second repertoires which together participate in a two or more step enzymatic reaction that creates the product from the substrate.

Such multi-step enzymatic reactions, in which the product of a first enzymatic reaction becomes the substrate for a second enzymatic reaction are known to those of skill in the are, along which enzymes which may be used in such reactions. Examples of multi-step enzyme reactions may be found, for example in U.S. Pat. Nos. 6,248,553 and 6,255,063.

The present invention still further provides a method for screening a plurality of cellular populations against a plurality of viral populations to identify those viral populations among the plurality of viral populations that infect cellular populations among the plurality of cellular populations, which method comprises:
(a) providing an array of members of the first repertoire juxtaposed with members of the second repertoire which permits interaction of the first repertoire members with the second repertoire members, the array comprising a solid surface, wherein the repertoire of cellular populations is present on a solid surface in a first series of continuous, non-intersecting lines such that each line of the series comprises a member of the repertoire of cellular populations, and the repertoire of viral populations is present on a solid surface in a second series of continuous, non-intersecting lines such that each line of the series comprises a member of the repertoire of viral populations, such that each of the first series of lines intersects with each of the second series of lines, such that members of the repertoire of cellular populations are juxtaposed to members of the repertoire of viral populations which permits interaction of the repertoire of cellular populations and the repertoire of the viral populations; and
(b) detecting viral infection in the plurality of cellular populations, thereby identifying viral populations among the repertoire of viral populations that infect cellular populations among the repertoire of cellular populations.

Methods for the detection of cellular infection are well known to those of skill in the art and include, for example, a variety of viability and cytotoxicity assays available from Molecular Probes (Eugene, OR). Such assays are suitable for detecting cell death and/or cell health in a variety of different cell types.

In another embodiment, the present invention relates to a method for screening a plurality of different cellular fractions against one another to identify those cellular fractions that contain components which interact with components in the other cellular fractions, which method comprises:
(a) providing an array, comprising a solid surface, comprising a first repertoire of cellular fractions and a second repertoire of cellular fractions wherein the first repertoire is present on a solid surface in a first series of continuous, non-intersecting lines such that each line of the series comprises an individual cell fraction of the first repertoire, and the second repertoire is present on a solid surface in a second series of continuous, non-intersecting lines such that each line of the series comprises an individual cell fraction of the second repertoire, such that each of the first series of lines intersects with each of the second series of lines, such that cell fractions of the first repertoire are juxtaposed to cell fractions of the second repertoire which permits interaction of the first and second repertoire cellular fractions; and
(b) detecting the interaction of different cellular fractions at sites where the different cellular fractions are juxtaposed, thereby identifying cellular fractions of the plurality that contain components which interact with components in other cellular fractions of the plurality.

Cellular fractions may be prepared using methods known to those of skill in the art such as those taught in Cell Biology A Laboratory Handbook (Academic Press 1994 *Editor J. E. Celis* ISBN 0-12-164715-3), and arrayed in a series of two or more repertoires according to the methods taught herein.

It is well known in the art that many cell types express cell-surface proteins which interact directly or through intermediate means with cell-surface proteins present on similar or dissimilar cell types. Such interactions play myriad roles in diverse physiological systems such as cellular adhesion, extracellular matrix interaction, and cell-cell communication, for example.

Accordingly, the present invention further provides a method for screening a plurality of different cellular populations against one another to identify those cellular populations that interact with the other cellular populations, which method comprises:
(a) providing an array, comprising a solid surface, comprising a first repertoire of cellular populations and a second repertoire of cellular populations wherein the first repertoire is present on a solid surface in a first series of continuous, non-intersecting lines such that each line of the series comprises an individual cell population of the first repertoire, and the second repertoire is present on a solid surface in a second series of continuous, non-intersecting lines such that each line of the series comprises an individual cell population of the second repertoire, such that each of the first series of lines intersects with each of the second series of lines, such that cell populations of the first repertoire are juxtaposed to cell populations of the second repertoire which permits interaction of the first and second repertoire cellular populations; and
(b) detecting the interaction of different cellular populations at sites where the different cellular populations are juxtaposed, thereby identifying cellular populations of the plurality that interact with other cellular populations of the plurality.

The invention also provides a method for screening each member of a polypeptide repertoire against each other member of the polypeptide repertoire, in order to identify members of the polypeptide repertoire that interact with other members of the polypeptide repertoire, which method comprises:
(a) providing an array, comprising a solid surface, comprising a first repertoire of polypeptides and a second repertoire of the same polypeptides wherein the first repertoire is present on a solid surface in a first series of continuous, non-intersecting lines such that each line of the series comprises a member of the first repertoire, and the second repertoire is present on a solid surface in a second series of continuous, non-intersecting lines such that each line of the series comprises a member of the second repertoire, such that each of the first series of lines intersects with each of the second series of lines, such that polypeptides of the first repertoire are juxtaposed to polypeptides of the second repertoire which permits interaction of the first and second repertoire polypeptides; and
(b) detecting the interaction of different members of the polypeptide repertoire at sites where the different members are juxtaposed, thereby identifying members of the polypeptide repertoire that interact with other members of the polypeptide repertoire.

In one embodiment, this is performed using a yeast two-hybrid system to identify those members of the repertoires of molecules that interact with one another.

The invention further provides a method for creating a combinatorial library comprising members of a first repertoire of polypeptides paired with members of a second repertoire of polypeptides, which method comprises:
(a) providing an array comprising a solid surface wherein a plurality of host cells comprising a plurality of nucleotide sequences encoding a first repertoire of polypeptide members is present on a solid surface in a first series of continuous, non-intersecting lines such that each line of the series comprises a member of the first repertoire of polypeptide members, and a plurality of nucleotide sequences encoding a second repertoire of polypeptide members is present on a solid surface in a second series of continuous, non-intersecting lines such that each line of the series comprises a member of the second repertoire of polypeptide sequences of to create an array, such that each of the first series of lines intersects with each of the second series; and
(b) transforming the cells containing the nucleotide members of the first repertoire with the nucleotide sequences that encode the members of the second repertoire where the two repertoires intersect; and
(c) expressing the nucleotide sequences to produce the corresponding polypeptides of the first and second repertoires; thereby creating a combinatorial library consisting of members of the first repertoire of polypeptides paired with members of the second repertoire of polypeptides.

According to the invention, a combinatorial library constructed in this manner may be screened for members of the first repertoire that interact with members of the second repertoire by a method comprising the step of detecting an interaction between the polypeptide members of the first and second repertoires, thereby identifying members of the first repertoire that interact with members of the second repertoire.

In addition the present invention relates to a method for screening the combinatorial library for members of a first repertoire of polypeptides that interact with members of a second repertoire of polypeptides, wherein the combinatorial library is generated by a method comprising providing an array comprising a solid surface and a plurality of host cells comprising a plurality of nucleotide sequences encoding the first repertoire of polypeptide members present on a solid surface in a first series of continuous, non-intersecting lines such that each line of the series comprises a member of the first repertoire of polypeptide members, and a plurality of nucleotide sequences encoding the second repertoire of polypeptide members present on a solid surface in a second series of continuous, non-intersecting lines such that each line of the series comprises a member of the second repertoire of polypeptide sequences of to create an array, such that each of the first series of lines intersects with each of the second series; transforming the cells containing the nucleotide members of the first repertoire with the nucleotide sequences that encode the members of the second repertoire where the two repertoires intersect; and expressing the nucleotide sequences to produce the corresponding polypeptides of the first and second repertoires; thereby creating a combinatorial library consisting of members of the first repertoire of polypeptides paired with members of the second repertoire of polypeptides, the method comprising the step of detecting an interaction between the polypeptide members of the first and second repertoires, thereby identifying members of the first repertoire that interact with members of the second repertoire.

The invention still further provides a method for creating a combinatorial library consisting of members of a first repertoire of polypeptides paired with members of a second repertoire of polypeptides, which method comprises:
(a) providing an array comprising a solid surface and a plurality of host cells comprising a plurality of nucleotide sequences encoding a first repertoire of polypeptide members present on a solid surface in a first series of continuous, non-intersecting lines such that each line of the series comprises a member of the first repertoire of polypeptide members, and a plurality of viruses containing a plurality of nucleotide sequences encoding a second repertoire of polypeptide members present on a solid surface in a second series of continuous, non-intersecting lines such that each line of the series comprises a member of the second repertoire of polypeptide sequences of to create an array, such that each of the first series of lines intersects with each of the second series;
(b) infecting the cells containing the nucleotide members encoding the first repertoire with the viruses that contain the nucleotide members encoding the second repertoire where the first and second series of lines intersect; and
(c) expressing the nucleotide sequences to produce the corresponding polypeptides of the first and second repertoires, thereby creating a combinatorial library consisting of members of the first repertoire of polypeptides paired with members of the second repertoire of polypeptides.

The invention further relates to a method of screening the combinatorial library to identify members of a first repertoire of polypeptides that interact with members of a second repertoire of polypeptides, wherein the combinatorial library is generated by the method comprising providing an array comprising a solid surface and a plurality of host cells comprising a plurality of nucleotide sequences encoding the first repertoire of polypeptide members present on a solid surface in a first series of continuous, non-intersecting lines such that each line of the series comprises a member of the first repertoire of polypeptide members, and a plurality of viruses containing a plurality of nucleotide sequences encoding the second repertoire of polypeptide members present on a solid surface in a second series of continuous, non-intersecting lines such that each line of the series comprises a member of the second repertoire of polypeptide sequences of to create an array, such that each of the first series of lines intersects with each of the second series; infecting the cells containing the nucleotide members encoding the first repertoire with the viruses that contain the nucleotide members encoding the second repertoire where the first and second series of lines intersect; and expressing the nucleotide sequences to produce the corresponding polypeptides of the first and second repertoires, thereby creating a combinatorial library consisting of members of the first repertoire of polypeptides paired with members of the second repertoire of polypeptides, the method comprising the step of detecting an interaction between polypeptide members of the first and second repertoires, whereby members of the first repertoire that interact with members of the second repertoire are identified.

The invention still further provides a method for creating a yeast two hybrid library consisting of members of a first repertoire of polypeptides paired with members of a second repertoire of polypeptides, which method comprises:
(a) providing an array comprising a solid surface and a first plurality of yeast cells comprising a plurality of nucleotide sequences encoding a first repertoire of polypeptide members present on a solid surface in a first series of continuous, non-intersecting lines such that each line of the series comprises a member of the first repertoire of polypeptide members, and a second plurality of yeast cells containing a plurality of nucleotide sequences encoding a second repertoire of polypeptide members present on a solid surface in a second series of continuous, non-intersecting lines such that each line of the series comprises a member of the second repertoire of polypeptide sequences of to create an array, such that each of the first series of lines intersects with each of the second series;
(b) allowing the yeast cells containing the members of the first repertoire to mate with the yeast cells containing the members of the second repertoire where the two repertoires intersect; and
(c) expressing the nucleotide sequences to produce the corresponding polypeptides of the first and second repertoires, thereby creating a yeast two hybrid library comprising members of a first repertoire of polypeptides paired with members of a second repertoire of polypeptides.

Methods for mating yeast cells are known in the art and may readily be adapted to the matrix screening assays described herein.

In one embodiment, the invention provides a method of screening a combinatorial library to identify members of a first repertoire of polypeptides that interact with members of a second repertoire of polypeptides, wherein the combinatorial library is generated by the method comprising providing an array comprising a solid surface and a first plurality of yeast cells comprising a plurality of nucleotide sequences encoding the first repertoire of polypeptide members present on a solid surface in a first series of continuous, non-intersecting lines such that each line of the series comprises a member of the first repertoire of polypeptide members, and a second plurality of yeast cells containing a plurality of nucleotide sequences encoding the second repertoire of polypeptide members present on a solid surface in a second series of continuous, non-intersecting lines such that each line of the series comprises a member of the second repertoire of polypeptide sequences of to create an array, such that each of the first series of lines intersects with each of the second series; allowing the yeast cells containing the members of the first repertoire to mate with the yeast cells containing the members of the second repertoire where the two repertoires intersect; and expressing the nucleotide sequences to produce the corresponding polypeptides of the first and second repertoires, thereby creating a yeast two hybrid library comprising members of the first repertoire of polypeptides paired with members of the second repertoire of polypeptides, the method comprising the step of detecting an interaction between the polypeptide members of the first and second repertoires, whereby members of the first repertoire that interact with members of the second repertoire are identified.

In a further embodiment, the invention provides a method of creating a combinatorial chemical library comprising:
(a) providing an array comprising a solid surface and a first repertoire of chemical groups comprising a first reactive group present on the solid surface in a first series of continuous, non-intersecting lines;
(b) reacting the solid surface with a reagent to modify the first reactive group to render the first reactive group capable of forming a chemical bond with a second reactive group;
(c) depositing a second repertoire on the solid surface comprising a second reactive group capable of forming a chemical bond with the first reactive group, wherein the second repertoire is deposited in a second series of continuous, non-intersecting lines, such that each line of the first series intersects with each line of the second series, such that each member of the first repertoire is juxtaposed to each member of the second repertoire, wherein a reactive group of the second repertoire forms a chemical bond with a reactive group of the second repertoire thereby producing a combinatorial chemical library.

The invention still further relates to a method for screening a first repertoire comprising a combinatorial chemical library against a repertoire of members to identify members of the first repertoire which interact with members of the second repertoire, wherein the combinatorial chemical library is produced by a method comprising providing an array comprising a solid surface and a first repertoire of chemical groups comprising a first reactive group present on the solid surface in a first series of continuous, non-intersecting lines; reacting the solid surface with a reagent to modify the first reactive group to render the first reactive group capable of forming a chemical bond with a second reactive group; depositing a second repertoire on the solid surface comprising a second reactive group capable of forming a chemical bond with the first reactive group, wherein the second repertoire is deposited in a second series of continuous, non-intersecting lines, such that each line of the first series intersects with each line of the second series, such that each member of the first repertoire is juxtaposed to each member of the second repertoire, wherein a reactive group of the second repertoire forms a chemical bond with a reactive group of the second repertoire thereby producing a combinatorial chemical library, the method comprising the step of juxtaposing the combinatorial chemical library to the second repertoire and detecting an interaction between members of the combinatorial library and members of the second repertoire, thereby identifying members of the first repertoire comprising the combinatorial library which interact with members of the second repertoire.

The method of the present invention bridges the gap between the initial identification of lead targets and molecules from very large repertoires and the final identification of targets or drugs for therapeutic intervention. This problem is addressed in the prior art by use of ELISA screening of possible positive interactants. However, protocols for ELISA are not easily automated for high throughput. The highly parallel nature of the method according to the present invention will reveal comprehensive interaction profiles for members of each repertoire. This will enable, for example, ligands that interact with an entire family of proteins to be distinguished form those which react with only a subset of that family, cross-reactive drugs to be eliminated from development programmes, and the true specificity and cross-reactivity of antibodies to be determined. The determination of an antibodies cross-reactivity and hence its specificity is of vital importance where there is a panel of different antibodies have been derived from an immunized mouse or from an *in vitro* selections performed, for example, by phage display. Matrix Screening is particularly powerful in this context as it enables a comprehensive range of antigens to be tested against each antibody in the panel, minimising the chance of unknown and unwanted cross reactivities disrupting downstream investigations.

Alternatively, by using the present invention to create and screen large comprehensively combinatorial libraries, one million clone antibody libraries could be created and screened using only 2,000 dispensing events. In addition, complex protein-protein interaction maps can be created from enriched sources of interacting pairs, or possibly using entire proteomes together with very high density matrices according to the invention.

The invention also incorporates the key advantages of phage display and other expression-display techniques, namely that the nucleic acids encoding the members of a polypeptide repertoire can be spatially associated with their corresponding polypeptides and can thus be selected on the basis of the functional characteristics of the individual polypeptide. Unlike phage display, however, in which this association is achieved by compartmentalising the nucleic acids and the polypeptides using bacterial cells which display the polypeptides on their surfaces, the subject invention advantageously exploits a novel arraying strategy to provide this association. By eliminating the requirement for the nucleic acids and the polypeptides to be retained in or on bacterial cells, the present invention can be extended beyond selection of binding activities to select any polypeptide repertoire on the basis of any functional property of the polypeptides, including enzymatic activity, conformation or any other detectable characteristic.

Various apparatus can be supplied in association with reagents or tools for performing the screens described above.

### Definitions

The term "repertoire" as used according to the present invention refers to a group of members, and also refers more narrowly to a group of members that share a common characteristic, such as a group of cells, a group of microorganisms, a group of proteins, a group of viruses, a group of nucleic acids, and a group of chemicals. More narrowly still, a "repertoire" may refer to variants of a particular type of member, that is, for example, a group of polynucleotide molecules, the group being based on a sequence which is mutated at specific positions to create variants of the basic sequence. Other examples of variants include, but are not limited to polypeptide sequence variants, cells which have been modified to express, for example, variant cell surface proteins which are derived from the same protein sequence, virus particles which have been modified to express variant envelope proteins which are derived from the same protein sequence, etc. Other members which may be used in a repertoire include cell type variants, nucleic acid sequence variants, virus strain variants, cellular fraction variants, small molecule variants, and the like. Generally, a repertoire includes more than 10 different variants. Large repertoires comprise the highest number of possible variants for selection and can be up to 10¹³ in size. Smaller repertoires are particularly useful, especially if they have been pre-selected to enrich for a particularly useful subset (for example, antibodies that bind cell surface markers, enzymes that catalyse a certain set of reactions, proteins that bind to other proteins etc) or to remove unwanted members (such as those including stop codons, incapable of correct folding or which are otherwise inactive). Such smaller repertories can comprise 10, 10², 10³, 10⁴, 10⁵, 10⁶ or more polypeptides. Advantageously, smaller repertoires comprise between 10 and 10⁴ polypeptides.

In the present invention, two or more repertoires of, for example, polypeptides are screened against each other. Advantageously, at lest 50% of the members or each repertoire are screened against each other in each screen. Preferably, 60%, 70%, 80%, 90%, 95% or even 100% of the members of each repertoire are so screened.

As used herein, the term "line" refers to a continuous distribution of an individual member of a repertoire having the physical shape of a line. A line as used herein may refer to a stream of an aqueous solution which is applied to a solid surface so as to form a line of solution. A line may alternatively refer to a series of droplets which are placed on a solid surface, and which coalesce to form a continuous line of solution. A line may alternatively refer to a solution or anhydrous compound which is deposited on a solid surface as a spray, provided that the solution or anhydrous compound is deposited in the form of a line. A line may also refer to a tube with a lumen into which a member of a repertoire useful in the invention is placed. A line, useful in the present invention is preferably XX long and YY wide A line may also refer to a groove, or channel which is cut into a solid surface, such as by manually scratching the surface, or by automated means such as laser etching. As used herein, "cut" refers to producing a linear indentation in a solid surface by scratching, etching, depressing, deforming, chipping, or gouging the solid surface.

As used herein, a "series" of lines refers to a plurality of lines each of which comprises an individual member of a repertoire. A plurality refers to at least 10 elements such as lines, members of a repertoire, etc., 10², 10³, 10⁴, 10⁵, 10⁶, and up to 10¹³ elements.

As used herein, "non-intersecting lines" refers to a series of lines in which no line in the series intersects, or contacts any other line in the same series.

As used herein, "solid surface" refers to a substrate which may have steps, ridges, kinks, terraces, and the like without ceasing to be a solid surface. A "solid surface" may further refer to a semi-solid surface such as a gel. As used herein, "semi-solid" refers to a compressible surface with a solid and liquid or gas component, wherein the liquid or gas occupies pores, spaces or other intersticies between the solid matrix elements. A "solid surface" may be a planar surface, or may alternatively be cuboidal, round, elliptical, or irregularly shaped. A "solid surface" as used herein may refer to a substrate comprising a single layer, or may refer to a substrate comprising multiple layers stacked on one another. "Solid surfaces" useful in the present invention include, but are not limited to glass, poly-styrene, nitrocellulose, PVDF filter, hydrogel, poly-carbonate or other plastic polymer slides, poly-styrene, poly-carbonate or other plastic polymer well plates, beads, membranes, glass wool, and other solid support materials for combinatorial chemistry reactions.

In the context of the present invention, "interact" refers to any detectable interaction between the molecules which comprise the various repertoires and, optionally, any additional molecules that comprise the screen. For example, in the case of antibody-antigen interactions one repertoire might comprise a diverse population of antibodies and the other a diverse population of antigens, the interaction being a binding interaction. Alternatively, the interaction can be an enzymatically-catalysed reaction, in which one repertoire is composed of enzymes and the other repertoire is composed of substrates therefor. Any interaction can be assayed using the present invention, including binding interactions, DNA methylation, nucleic acid degradation, nucleic acid cleavage (single or double stranded), signalling events, catalytic reactions, phosphorylation events, glycosylation events, proteolytic cleavage, chemical reactions, cellular infection and combinations thereof. The detection of such interactions is well known in the art.

In the context of the present invention, "molecule" refers to any substance which can be applied to the screen. Such molecules can include peptides, polypeptides, nucleic acid molecules, purified proteins, recombinant proteins, amino acids, cDNAs, expressed cDNAs, oligonucleotides, nucleotides, nucleotide analogues, families of related genes or the corresponding proteins thereof, enzymes, DNA binding proteins, immunoglobulin family members, antibodies, T cell receptors, haptens, small organic molecules, non-organic compounds, metal ions, carbohydrates and combinations thereof. The creation of repertoires of such molecules is well know in the art. A repertoire may originate from a single molecules or variants of that molecule. As used herein, a "molecule" can refer to a "small molecule", wherein a "small molecule" refers to a compound which is not itself the product of gene transcription or translation (protein, RNA or DNA). Preferably a "small molecule" is a low molecular weight compound, e.g., less than 7500 amu, more preferably less 5000 amu and even more preferably less than 2500 amu. Examples of small molecules include, among the many compounds commonly referred to as "natural products", beta-lactam antibiotics, steroids, retinoids, polyketides, etc.
The combinatorial production of collections of non-oligomeric, small molecule compounds has been described (DeWitt et al., Proc. Natl. Acad. Sci., USA 90:690, 1993; Bunin et al., Proc. Natl. Acad. Sci., USA 91:4708, 1994). Structures suitable for elaboration into small-molecule libraries encompass a wide variety of organic molecules, for example heterocyclics, aromatics, alicyclics, aliphatics, steroids, antibiotics, enzyme inhibitors, ligands, hormones, drugs, alkaloids, opioids, terpenes, porphyrins, toxins, catalysts, as well as combinations thereof.

"Polypeptides" can refer to polypeptides such as expressed cDNAs, members of the immunoglobulin superfamily, such as antibody polypeptides or T-cell receptor polypeptides. Advantageously, antibody repertoires can comprise repertoires comprising both heavy chain (V_{H}) and light chain (V_{L}) polypeptides, which are either pre-assembled or assembled and screened according to the present invention. An antibody polypeptide, as used herein, is a polypeptide which either is an antibody or is a part of an antibody, modified or unmodified. Thus, the term antibody polypeptide includes a heavy chain, a light chain, a heavy chain-light chain dimer, a Fab fragment, a F(ab')₂ fragment, a Dab fragment, a light or heavy chain single domain, and an Fv fragment, including a single chain Fv (scFv), linked single domains such as V_{H}-V_{H} or V_{L}-V_{L}, or a di-sulphide bonded Fv (dsFv). Methods for the construction of such antibody molecules and nucleic acids encoding them are well known in the art. However, "polypeptides" can refer to other polypeptides, such as enzymes, antigens, drugs, molecules involved in cell signalling, such as receptor molecules, or one or more individual domains of larger polypeptides, which are capable of an interaction with a target molecule. As used herein, "polypeptide" may also refer to a peptide. Molecules according to the invention can be provided in cellular form, that is in the form of cells producing a molecule as described above, or in non-cellular form, that is not contained within cells. Cells can be, for example, bacterial cells, lower eukaryotic cells (e.g., yeasts), or higher eukaryotic cells (e.g., insect, amphibian, avian or mammalian cells). In one embodiment, the cells may be cells obtained from a patient, such as blood cells, organ cells, lymphoid cells, tumour cells, nerve cells, and the like.

Many of the "molecules" of the present invention may be referred to as "biomolecules", wherein a "biomolecule" refers to a molecule found within or made by an organism in nature. The term "biomolecule" also refers to chemically modified or synthetic forms of molecules corresponding to those found within or made by an organism in nature. Biomolecules of interest include, but are not limited to, nucleic acids (oligonucleotides or polynucleotides of DNA, RNA or PNA), peptides, polypeptides, proteins, and antibodies.
In the context of the present invention, the term "cellular population" refers to a collection of cells. The cells comprising a cellular population may all be of the same species and cell type, or they may be a mixed population. One embodiment of a cellular population comprises an essentially substantially uniform population of cells, for example mammalian fibroblasts, transformed with a library encoding variants of a given gene coding sequence.

In the context of the present invention, the term "viral population" refers to a collection of virus particles. The particles comprising a viral population may all be of the same species and strain, or they may be a mixed population. One embodiment of a viral population comprises population of recombinant or randomly mutagenized particles, for example retroviral particles. A viral population can comprise multiple individuals carrying variations of one or more gene coding sequences.

"Juxtaposition", in the context of the present invention, includes but is not limited to physical contact. Two or more repertoires according to the invention can be juxtaposed such that the molecules are capable of interacting with one another in such a manner that the sites of interactions between the members of the repertoires can be correlated with their position. Alternatively, the repertoires can be juxtaposed with one another and with a target molecule such that the members of the repertoires interact with one another and then together interact with a target molecule. According to the present invention, repertoires are "juxtaposed" if they are separated by no more that 20 µm, preferably no more than 10 µm, and still more preferably no more than 5 µm. Preferably, two or more repertoires are "juxtaposed" by the intersection of lines which comprise two or more members of the repertoires, wherein the lines intersect at between a 1° and 179° angle, preferably between a 45° and 135° angle, and more preferably at a 90° angle.

An "array" as referred to herein, is a pre-determined spatial arrangement of the members of the repertoire wherein the array comprises a solid surface on which at least the members of a first repertoire are applied in a series of continuous, non-intersecting lines, and wherein each line comprises a member of the repertoire. An "array" can also refer to a solid surface on which is present the members of a first repertoire in a first series of continuous, non-intersecting lines and the members of a second repertoire in a second series of continuous, non-intersecting lines, provides that each of the first series of lines intersects with each of the second series of lines. The array can take any physical form, but preferably comprises one solid surface on which is applied two or more repertoires in a series of continuous, non-intersecting lines, or may comprise two or more solid surfaces on each of which is applied a repertoire in a series of continuous, non-intersecting lines, provided that within the array, every member of one repertoire is juxtaposed to every member of a second repertoire (e.g., in a two-dimensional format) (and/or third repertoire; e.g., in a three-dimensional format). The array can be created by manual or automated means and preferred arraying technologies are further described below. Alternatively, an "array" may refer to a first repertoire present in the lumen of a first series of non-intersecting tubes, wherein each tube of the first series comprises a member of the first repertoire, and a second repertoire present in the lumen of a second series of non-intersecting tubes, wherein each tube of the second series comprises a member of the second repertoire, and wherein each of the first series of tubes intersects with each of the second series of tubes. An "array" as used herein may take the form of, for example, two or more intersecting sets of parallel lines, tubes, or channels, a series of concentric circles intersected by a plurality of radially disposed lines, or a spiral intersected by a plurality of radially disposed lines. An "array" as used herein may further refer to two or more series of non-intersecting lines, wherein each of the lines of a first series intersects with each of the lines of a second series, and wherein each of the series of lines is present as a anhydrous or fluid line between two opposed planar solid surfaces.

A "matrix" in the context of the present invention, may be used interchangeably with the term "array". A matrix can be used to study all possible interactions between all the members in two or more repertoires of molecules. Such matrices, as used herein can comprise a series of intersecting lines, channels or tubes, each containing one or more members of the repertoires which are present in a series of continuous, non-intersecting lines, channels or tubes. A single matrix will contain many individual lines, channels or tubes and many more intersections, or nodes.

As used herein, "continuous" as it refers to a non-intersecting line, refers to the fact that an individual member of a repertoire on an array is present along the entire length of the line, that is, there should be no position in line where the individual member of the repertoire is not present, although the density or concentration of the member may vary along the length of the line.

A "dispensing event" is a single event whereby a substance is transferred from one discrete location to a second discrete location. A discrete location can be in the form of a well, a tube, a channel, a spot, a line, a rectangle, a sphere, a cube etc. Examples of single dispensing events include:
(i) pipetting a liquid from one tube or well to a second tube or well. In this case pipetting aliquots of the same liquid into multiple tubes or wells would be considered to be multiple dispensing events, as would dispensing two or more different liquids into the same tube or well. or
(ii) transferring liquid from a source well to a membrane by pin transfer to create a spot of that liquid. In this case spotting a second aliquot from the same source well onto a different destination location on the membrane would be considered a separate dispensing event, or
(iii) transferring liquid from a single source well to create a single continuous line of liquid on a membrane. In this case creating a second separate line, even of the same liquid, would be considered a separate dispensing event, or
(iv) dispensing a solution down a tube or channel. In this case, dispensing a different solution down the same tube or channel, or the same solution down a different tube or channel would be considered a separate dispensing event.

As used herein, the term "depositing" refers to the step of placing a member of a repertoire of the present invention on a solid surface, such that the member becomes stably associated with the surface. Wherein "stably associated" refers to a repertoire member that is bound to the solid substrate to form an array via covalent bonds, hydrogen bonds or ionic interactions, or protein/protein interactions, such as antigen/antibody interactions, such that the repertoire member retains its unique pre-selected position relative to all other repertoire members that are stably associated with an array, or to all other pre-selected regions on the solid substrate under conditions in which an array is typically analyzed (i.e., during one or more steps of hybridization, washes, and/or scanning, and the like). As used herein, "depositing" may also refer to the placement of a repertoire member into the lumen of a tube or into a channel present in a solid surface.

The term "enhanced" as used herein means that a detected interaction is increased by at least 10% in the presence of a given molecule or molecules relative to the interaction in the absence of that molecule or molecules.

The term "blocked" as used herein means that a detected interaction is decreased by at least 10% in the presence of a given molecule or molecules relative to the interaction in the absence of that molecule or molecules.

The term "optimizing", as used herein, refers to a process of testing different reaction parameters including, but not limited to pH, concentration, temperature, viscosity, ionic strength, buffer composition, a substrate concentration, the presence of denaturants, the presence of renaturants to determine which reaction parameters permits a desired level of interaction, and selecting the reaction parameters which either enhance or block the level of interaction.

The term "positive interaction", as used herein, refers to an interaction between two or more members of two or more arrayed repertoires, wherein the interaction generates a detectable signal (e.g., fluorogenic or chromogenic signal, cellular mortality, etc.).

The term "negative interaction", as used herein, refers to an interaction between two or more members of two or more arrayed repertoires, wherein the interaction does not generate a detectable signal (e.g., fluorogenic or chromogenic signal, cellular mortality, etc.).

The term "naked", as used herein in reference to "naked" DNA, RNA, or nucleic acid refers to nucleic acid which is substantially free (i.e., ≥ 98% by weight) from substances which facilitate entry of the nucleic acid into a host cell, for example, liposomes, ligands specific for cell surface receptors, endosomal disruption agents, etc.

The term "complexed", as used herein in reference to "complexed" DNA, RNA, or nucleic acid refers to nucleic acid which is not substantially free from substances which facilitate entry of the nucleic acid into a host cell, for example, liposomes, ligands specific for cell surface receptors, endosomal disruption agents, etc.

The term "cellular fraction" as used herein means a portion of a cell lysate resulting from a cell fractionation process. Non-limiting examples of cell fractionation processes include, detergent extraction, salt extraction, acid precipitation, extraction of lipid soluble components, membrane isolation, extraction of water soluble or aqueous components, nucleo/cytoplasmic fractionation, and separations based on centrifugal forces (e.g., the S-100 fraction). Other separations considered to be cell fractionation processes include nucleic acid isolation, chromatographic separation of components of cell lysate or fractionated cell lysate, preparative electrophoretic fractionation, ion exchange and affinity separations (e.g., immunoprecipitation or immunoaffinity chromatography, His/Ni++ interactions, GST/glutathione interactions, etc.).

### Brief Description of the Drawings

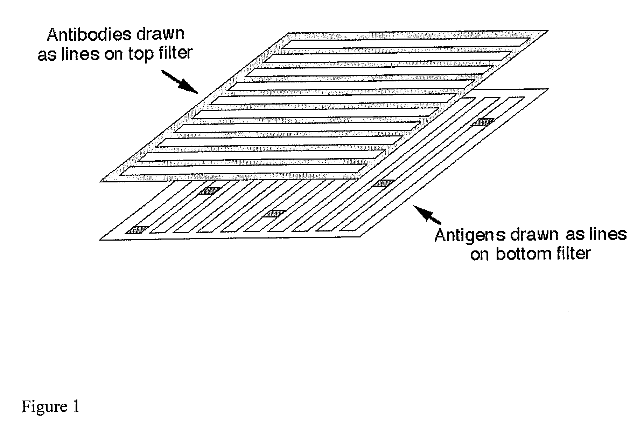

**Figure 1:** Outline of one method for screening a repertoire of antibodies against a repertoire of antigens according to the present invention, demonstrating how hundreds of different antibodies could be screened simultaneously against hundreds of different antigens to identify interacting pairs. Specific interactions are indicated.

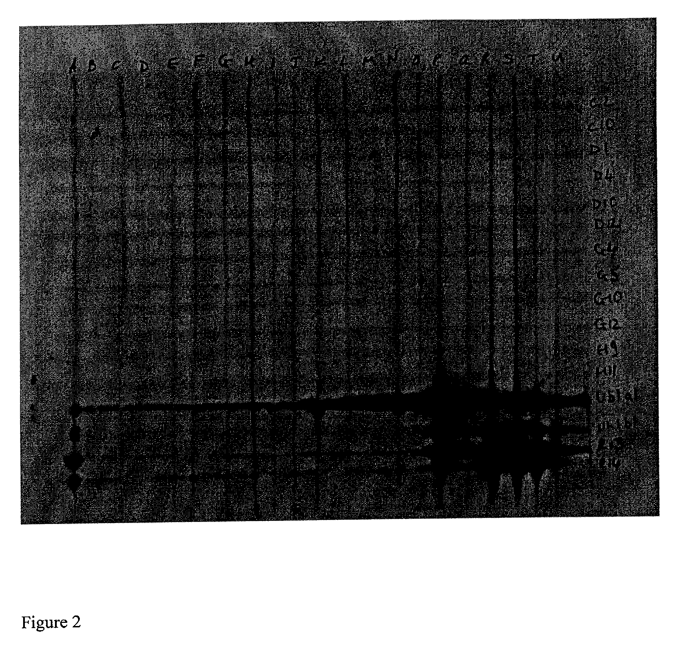

**Figure 2:** Analysis of scFvs using a manually created matrix. Here, 21 antigens (horizontally) are screened against 16 scFvs (vertically). Four scFvs have been selected against ubiquitin by phage selection (Ub1b1, Ub1a1, R13 and R14). Two antigen clones are known to be ubiquitin (Q and T) and five other clones (A, P, R, S and U) have been identified in a primary screen as probably binding an anti-ubiquitin scFv. Each of the four anti ubiquitin scFvs binds the two known ubiquitin clones and each of the five potential ubiquitin clones. However, it can be seen that scFv Ub1b1 and scFv R14 are highly cross reactive.

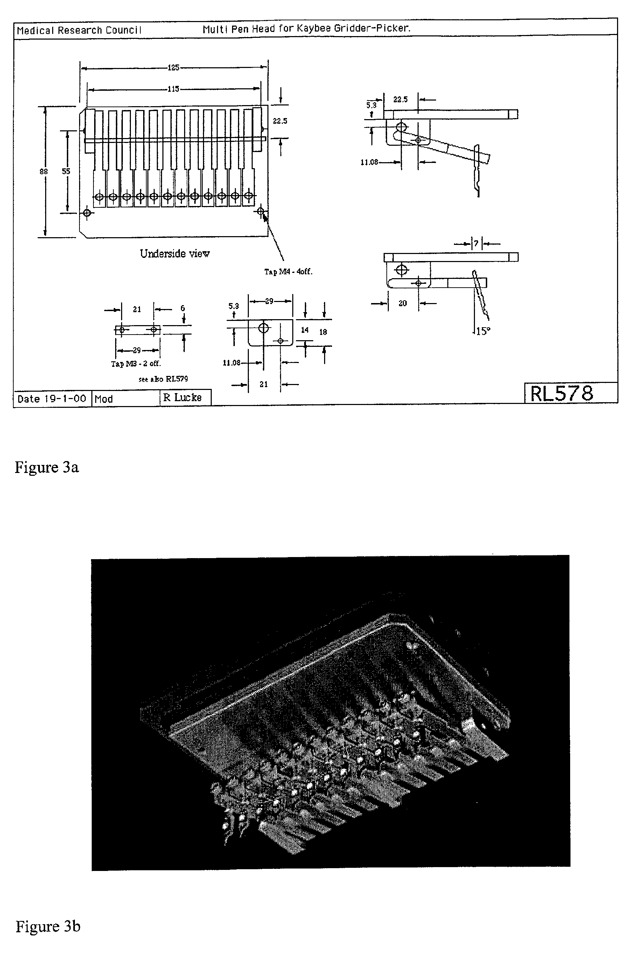

**Figure 3:** A head for robotic line drawing according to the present invention designed for mounting on a robotic platform which allows movement in x, y and z dimensions. A row of fountain pen nibs delivers repertoire members in a liquid suspension, by capillary action to a suitable solid support. The nibs are mounted in such a way as to deliver liquid at an optimum angle to the solid support and then to be held vertical by a stop for loading with liquid from a 96 well microtitre plate.

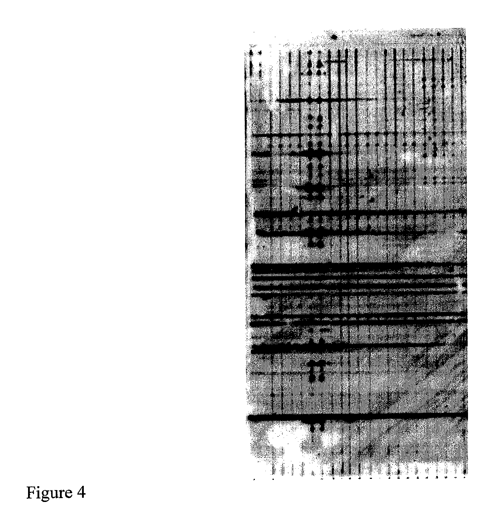

**Figure 4:** Analysis of scFvs using a robotically created matrix. Double lines of 12 antigens (horizontally) are screened against 192 scFvs (vertically). Specific interactions can be observed at the intersections of specific pairings. In addition, scFvs that cross-react with the nitrocellulose can be seen as continuous horizontal lines as can scFvs that cross-react with all antigens (horizontal spotting).

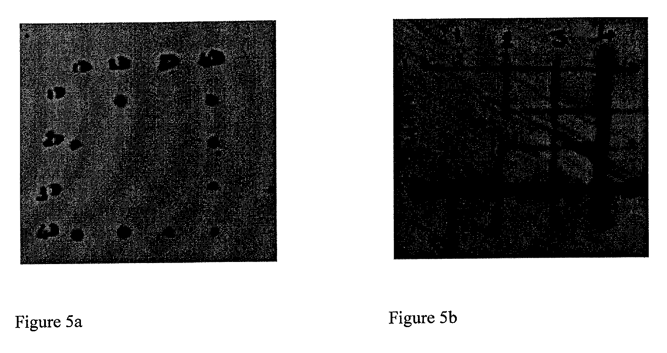

**Figure 5:** Example of creation and screening of a two-chain antibody repertoire. (a) A spotted array according to the prior art. Bacteria that secrete 1. Bovine Serum Albumin (BSA) binding heavy chains, 2. BSA binding light chains, 3. non binding heavy and light chains or 4. BSA binding heavy and light chains were mixed and then grown and induced in close proximity to immobilised BSA indicating that 1 and 2 need to be mixed to get a binding antibody (as seen in the control, 4). 32 separate dispensing events were required to produce this screen. (b) A matrix screen according to the present invention allows the same screen to be performed using only 8 dispensing events (the lack of a signal for 1 down with 2 across is probably due to a bubble being present between the filters during induction).

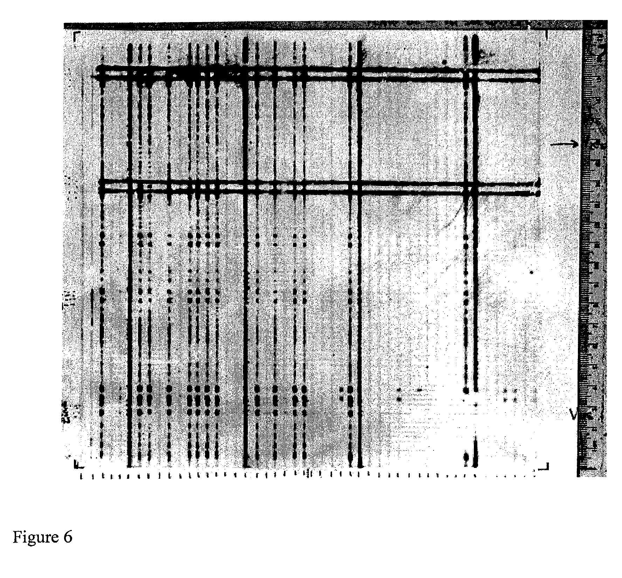

**Figure 6:** By increasing the density of the heavy and light chain lines higher density antibody arrays can be created and screened. Thus, 24 anti-BSA heavy chains and 48 anti-BSA light chains were drawn perpendicular to the x and y axes to create 1152 pairings screened against BSA.

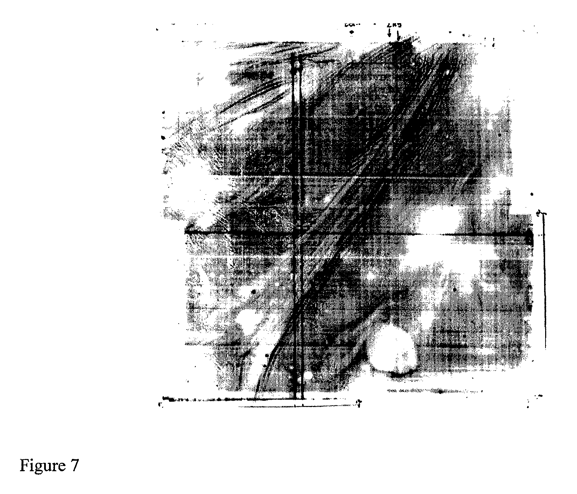

**Figure 7:** 384 unselected heavy chains and 384 unselected light chains were drawn perpendicular to the x and y axes and screened against BSA coated onto a nitrocellulose filter (147,456 combinations). A single specific heavy and light chain pairing was isolated which was subsequently confirmed as binding to nitrocellulose.

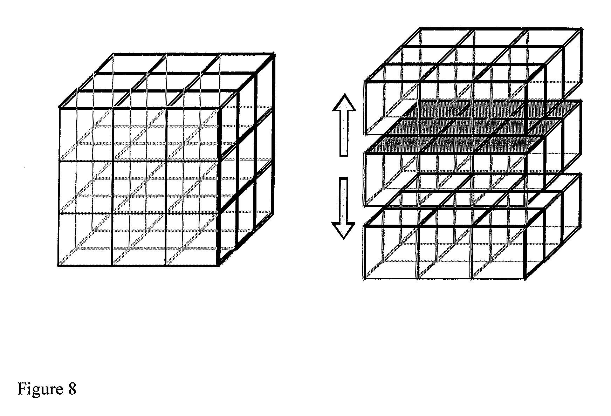

**Figure 8:** Schematic for three-dimensional screening according to the invention. Here, members of the repertoires are arranged in planes in the x, y and z axes and the interactions occur at the various vertices of the intersecting planes.

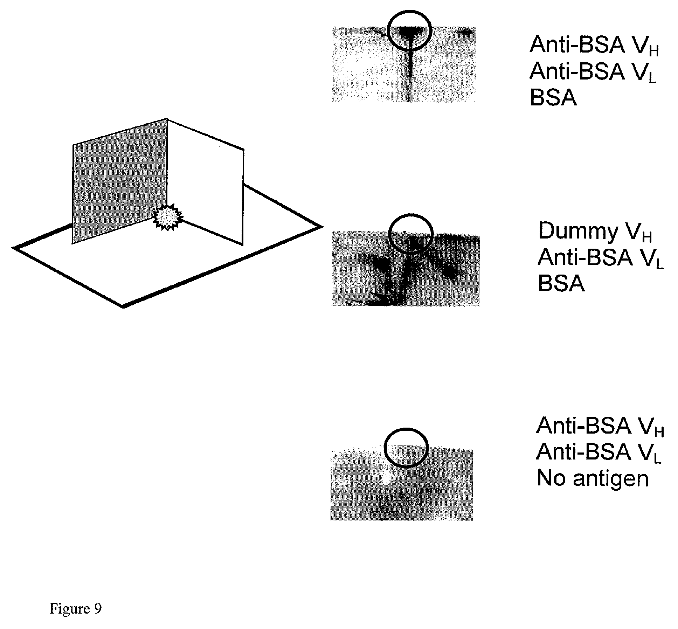

**Figure 9:** Proof of concept of a three-dimensional screen. Panel A shows a diagram of the three-dimensional screen. Panel B shows the anti-BSA V_{H}; anti-BSA V_{L}; BSA coated filter. Panel C shows a negative control anti-BSA V_{H}; anti-BSA V_{L}; BSA coated filter. Panel D shows a anti-BSA V_{H}; anti-BSA V_{L}; BSA marvel coated filter. The anti-BSA heavy chain is deposited on one plane, the anti-BSA light chain is deposited on a second plane, and BSA is deposited in the third plane. An interaction at their vertex is detected only when all three are present.

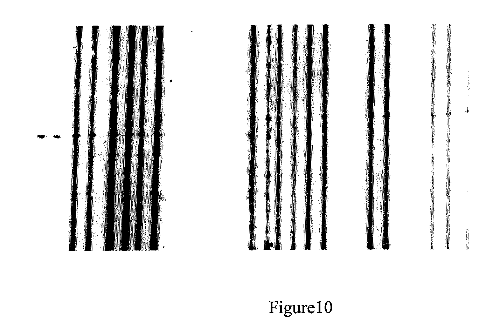

**Figure 10:** Using a robotically created two-dimensional array to analyse scFv-endogenous antigen binding. Double vertical lines of 48 recombinant scFvs are intersected with single horizontal lines of size-separated proteins from Bovine Mitochondrial complex I. One scFv, IA111 that is specific subunit 51 is detected. Several scFvs that cross-react with the nitrocellulose can be seen as continuous vertical lines.

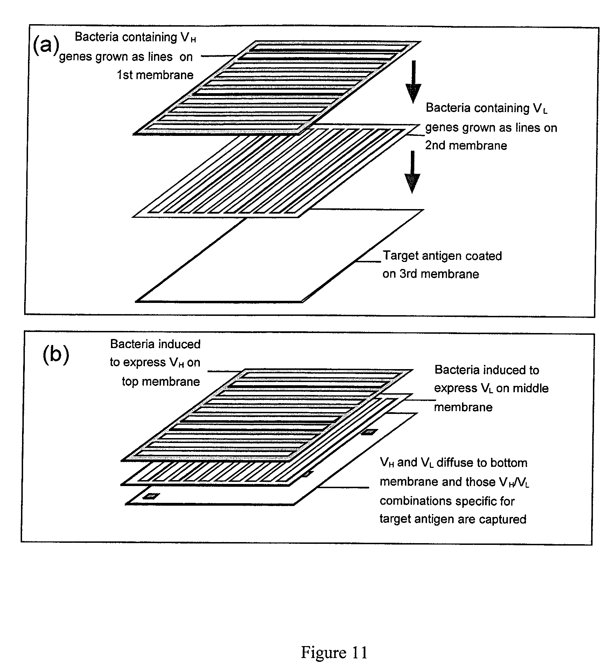

**Figure 11:** This schematic shows the method for creating of a repertoire of antibodies on a two-dimensional array. Lines of bacteria containing genes for recombinant antibody heavy chains (V_{H}) are grown on one membrane. Lines of bacteria containing genes for recombinant antibody light chains (V_{L}) are grown on a second membrane (a). The membranes are stacked one on top of the other (with their lines of bacteria at right angles) and placed on a third membrane coated in target antigen. Bacteria are then induced to express their antibody fragments and the V_{H} and V_{L} diffuse through the top two membranes to the antigen coated membrane (b). At intersections between lines, V_{H} and V_{L} associate and form a complete, active antigen-binding site. Those V_{H} and V_{L} combinations that do not bind antigen are washed away. Any V_{H} and V_{L} combinations that bind to the target antigen are captured on the antigen membrane and detected with Protein L-HRP.

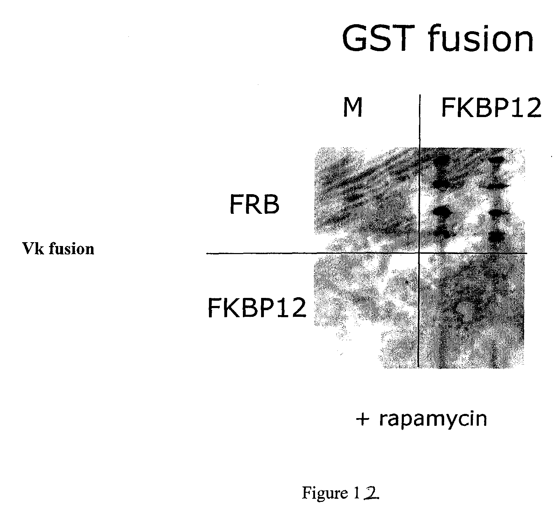

**Figure 12:** This figure shows the specificity of protein-protein interactions using matrix screening. Receptor GST FKBP-12 or GST-M are grown and expressed in the vertical direction and ligands V kappa FKBP-12 or V kappa FRB in the horizontal direction. Only those intersecting points where the cognate pairs are expressed can be detected (captured using anti-GST IgG, detected using protein-L-HRP). The interaction is strictly dependent on the presence of the mediator rapamycin.

### Detailed Description of the Invention

Matrices according to the present invention can be generated in many different ways to screen many different interactions involving many different molecules. The invention is characterised by the ability to screen all combinations of members of two or more repertoires. We have shown that this can be performed using a series of intersecting lines, but other approaches which allow combinatorial screening of two or more repertoires using a minimum number of dispensing events are envisaged, such as the use of intersecting channels or tubes. Our method relies on the juxtaposition of continuous groupings of molecules to create a web in two or three dimensions whereby members of the different repertoires come together and potentially interact with one another. This contrasts with screening protocols in the prior art, whereby discontinuous spotting or compartmentalised wells are used to segregate individual combinations of molecules. In the present invention, continuous deposits of individual members of a repertoire, in the form of lines, channels or tubes intersect one another such that individual combinations of molecules exist at their points of intersection, or nodes. Taken as a whole, the molecular 'web' or 'network' thereby created can be used not only to identify specific interacting pairs, but also the overall pattern of interactions between two repertoires. The information so provided can be used to compare the performance of members of either of the repertoires with one another and in particular can be used to rapidly identify cross-reactivities of individual repertoire members within the matrix.

### Repertoires for screening

The present invention relates to the screening of two or more repertoires of molecules. Repertoires of the present invention include, but are not limited to cells, protein, amino acids, nucleic acid, DNA, RNA, PNA, cellular fraction, virus, virus particles, small molecules, chemicals, enzymes, substrates, and antibodies. The many different repertoires which can be used with the present invention, may be constructed or obtained by methods well known by those skilled in the art. Repertoires of small organic molecules can be constructed by methods of combinatorial chemistry. Repertoires of peptides can be synthesised on a set of pins or rods, such as described in WO84/03564. A similar method involving peptide synthesis on beads, which forms a peptide repertoire in which each bead is an individual repertoire member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121. Although these repertoires could be constructed prior to arraying to produce the matrix, it is envisaged that all the techniques described above could be adapted for *in situ* synthesis of the repertoire members directly on the matrix itself - thus linking repertoire construction and repertoire screening according to the present invention. Indeed, another chemical synthesis method taught in the art involves the synthesis of arrays of peptides (or peptidomimetics) on a surface in a manner that places each distinct library member (e.g., unique peptide sequence) as a spot the array. The identity of each library member is therefore determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092; Fodor *et al*. (1991) *Science,* **251**: 767; Dower and Fodor (1991) *Ann. Rep. Med. Chem*., **26**: 271 and could be easily be adapted for creation of matrices according to the present invention.

The present invention is especially useful for the screening of protein-protein interactions, particularly antibody-antigen interactions. The preparation of appropriate antibody repertoires useful in the present invention is described in WO 99/20749, the disclosure of which is incorporated herein by reference. WO 99/20749 describes how a library of immunoglobulins can be prepared and preselected using a generic ligand, and/or prepared using a single main-chain conformation. Libraries as described in WO 99/20749 can be expressed in host organisms, as described therein or according to techniques well known in the art, to produce repertoires of polypeptides which are suitable for arraying and use in the present invention. Alternatively, polypeptides can be synthesised *in situ* for use in the present invention, or expressed using *in vitro* transcription/translation.

### Arraying members of the repertoires to create the matrix screen

According to the present invention, molecules can be arrayed by any one of a variety of methods, manual or automated, in order to create a matrix, depending upon whether the molecules are arrayed as such or expressed by arrayed nucleotide precursors, which may or may not be present in host cells.

Arrays useful in the present invention are constructed preferably on a solid surface such as glass, poly-styrene, poly-carbonate or other plastic polymer slides, poly-styrene, poly-carbonate or other plastic polymer well plates, beads, membranes, glass wool, and other solid support materials for combinatorial chemistry reactions. Briefly, members of a repertoire, such as nucleic acid variants suspended in a suitable buffer are deposited on a surface such as a nylon membrane in a series of parallel, continuous, non-intersecting lines, such that each member of the repertoire is deposited as a separate line. In one embodiment, members of a second repertoire are deposited as a second series of parallel, continuous, non-intersecting lines which are placed at a 90° angle to the orientation of the first series of lines, such that each line of the first series intersects with each line of the second series. In this manner each member of the first and second repertoires are juxtaposed to each other member.

Alternatively, the second repertoire may be deposited on a second solid surface in a series of parallel, continuous, non-intersecting lines. The second solid surface may then be placed in contact with the first solid surface such that the second series of lines is oriented perpendicular to the first series of lines so that each of the first series of lines intersects with each of the second series of lines. In this manner, each member of the first repertoire is juxtaposed to each member of the second repertoire.

In an alternate embodiment, the array may be produced in three dimensions. In one aspect of this embodiment, each of three repertoires may be arranged on a plane and the three sets of planes may be juxtaposed to provide an interaction of the members of each of the three repertoires at the vertex of the three planes. Alternatively, a three dimensional array may be produced by depositing members of a first, second and third repertoires in a first, second, and third series of lines, wherein each series of lines intersects with each other series of lines. For example, a first repertoire may be deposited in a gel such as agarose gel, or other solid or semi-solid surface by electrophoresis, injection, etc., such that the members of the repertoire are arrayed in a series of non-intersecting, continuous lines. A second repertoire may then be deposited into the same gel, but at an orientation which is perpendicular to the orientation of the first repertoire. For example, the members of the first repertoire may be injected into the gel along the X-axis, and the members of the second repertoire may be injected into the gel along the Y-axis. A third repertoire may then be injected into the same gel along the Z-axis at each point of intersection of the first and second repertoires, thus creating a three dimensional array. Alternatively, a three dimensional array may be generated by depositing a first repertoire as a series of continuous, non-intersecting lines on a first surface (such as a membrane) depositing a second repertoire as a series of continuous, non-intersecting lines on a second surface, and depositing a third repertoire in a series of continuous, non-intersecting lines on a third surface. The three surfaces may then be overlaid such that each of the first, second, and third series of lines intersect, and further such that each member of the first, second, and third repertoires is juxtaposed to each other member.

Arrays are advantageously created by robotic means, since robotic techniques allow the creation of precise and condensed matrices, which can be easily replicated so that, for example, a combinatorial antibody repertoire created according to the invention can be screened against many different target ligands. Robotic platforms are well-known in the art, and machines are available from companies such as Genetix, Genetic MicroSystems and BioRobotics which are capable of arraying at high speed with great accuracy over small or large surfaces. Such machines are capable of spotting purified protein, supernatant or cells onto porous or non-porous surfaces, such that they can subsequently be fixed thereto if necessary to produce stable arrays. Although robotic manipulation is the preferred method for creating high density arrays, any technique, including manual techniques, which is suitable for locating molecules or cells at pre-defined locations on a support, can be used.

Arraying can be regular, such that lines are 'drawn' at a given distance from the next, wherein "drawing a line" refers to depositing an individual member of a repertoire continuously in a linear manner, such that the member of the array is present at all points along the line. Arraying may be irregular, in that the distance between the lines, or the length of the lines may vary or the arraying can be random, following no discernible pattern or orientation, but provided that each member of a randomly arrayed first repertoire is juxtaposed to each member of an arrayed second repertoire. The lines (or tubes or channels) of the present invention which are used to construct the matrices described herein may be arranged in any orientation such that each member of a repertoire is juxtaposed to each other member of all other repertoires in the matrix. The repertoires may, for example, be arranged as two or more sets of intersecting parallel lines, a series of concentric circles intersected by a set of radially disposed lines, or a spiral intersected by a set of radially disposed lines. The matrices of the present invention are not limited however to two-dimensions. For example, a matrix array comprising two repertoires of intersecting parallel lines may be further intersected by a third set of lines oriented perpendicular to the first two repertoires as described above.

The repertoires of molecules can be screened in solution for interactions or one or more of the repertoires can be immobilised on a solid support. Thus, two solutions can flow down two channels such that at their point of intersection an interaction occurs which can be detected by, for example, a colorimetric, fluorescent, or luminescent reaction. Alternatively, one of the repertoires could be immobilised on a nitrocellulose membrane by, for example, cross-linking and then solutions corresponding to a second repertoire could be applied to the same support as a series of continuous, non-intersecting lines . Such immobilisation can be direct or indirect. For example, the a nucleic acid repertoire can be directly immobilized to a surface by cross-linking. Alternatively, a polypeptide, for example, could be labelled with a tag according to methods known in the art, wherein an antibody for the tag is immobilized on the surface, such that when the tag-labelled polypeptide is applied to the surface it is indirectly immobilized by way of its being bound by the antibody.

In one aspect the members of a repertoire may be deposited in the lumen of a series of non-intersecting tubes. Multiple repertoires in multiple series of tubes may then be intersected so as to juxtapose members of each repertoire with each other member of each repertoire. Types of tubing which are useful in the present invention are known to those of skill in the art and include, but are not limited to plastic tubing, rubber tubing, vinyl tubing, silicone tubing, Tygon® tubing, PVC tubing, fluoroelastomer tubing, PVDF tubing, polyethylene tubing, metal tubing and the like. In one embodiment, the tubing used is non-permeable. In this embodiment, intersections of tubes comprising members of two or more repertoires are achieved by using coupling means such as Luer fittings, stopcocks, barbed tubing connectors and the like (coupling means may be obtained from numerous vendors such as Harvard Apparatus, Holliston, MA). Coupling means may also encompass taping two or more ends of tubing together so as to achieve a patent connection between tubes, or fusing two or more tubes together by heating or welding.

Alternatively, tubes useful in the present invention may be permeable such as dialysis tubing, thus permitting members of a repertoire to defuse out of the entire length of the tubing. Thus, tubes from two or more repertoires may be contacted to one another such that the members of the repertoire are juxtaposed as they diffuse from the lumen of the permeable tubing and come into contact with members of another repertoire which have diffused from their respective permeable tubing. Permeable tubing may be obtained from numerous sources known to those of skill in the art including but not limited to BioDesign, Inc., Carmel, NY.

In one aspect, members of the repertoires are directed to their positions by means of a tagging system, such that each line, channel or tube binds or is predisposed to bind a particular member of the repertoire. For example, each polypeptide in one member of a repertoire can comprise a tag, such as an epitope for a known antibody, or a member of an affinity pair (e.g., avidin/biotin, etc.). The line, channel or tube is coated with one or more elements, such as a corresponding molecule that binds the tag (e.g., an antibody specific for the epitope tag, or the corresponding member of the binding pair). Contacting the coated line, channel or tube with a solution comprising the tagged member of the repertoire will effect the arrangement of that member on the array.

Alternatively, both repertoires could be immobilised on a separate solid supports and then juxtaposed to identify interacting pairs. In a preferred aspect of the invention, matrices of polypeptides can be created by first arraying their nucleic acid precursors in host cells and then by expressing the nucleotide sequences to produce the corresponding polypeptides.

In one aspect, yeast cells can be used to express one or more repertoires of molecules useful in a method according to the invention. Methods of introducing and expressing exogenous nucleic acids in yeast are well known in the art. One preferred approach using yeast takes advantage of yeast two-hybrid techniques. In this approach, one population of yeast is transformed with a library encoding a repertoire of fusions with one member of a two-hybrid pair, and another population is transformed with a library encoding a repertoire of fusions with the corresponding second member of a two-hybrid pair. The two yeast cell populations are of different mating types. The two populations are arranged so as to create an array, such that yeast cells containing all members of the first repertoire intersect with yeast cells containing all members of the second repertoire, and the cells are allowed to mate. Interactions between members of the first repertoire and the second repertoire will generate a signal from an appropriate two-hybrid reporter construct. The methodology underlying the yeast two-hybrid system, including expressing hybrid proteins in yeast and mating schemes, is widely known to those of skill in the art (See, for example, Schwikowski et al., 2000, *Nature Biotech*. 18: 1257-1261; Fields and Song, 1989, *Nature* 340: 245-246; Bendixen et al., 1994, *Nucleic Acids Research* 22: 1778-1779; Finley and Brent, 1994, *Proc Natl Acad Sci USA* 91: 12980-12984; Finley and Brent, 1995 DNA Cloning 2 Expression Systems: A Practical Approach, B. D. Hames and D. M. Glover, eds. (Oxford: Oxford University Press), pp. 169-203).

In another aspect, insect, amphibian, avian, mammalian or other higher eukaryotic cells can be used. As a non-limiting example, a repertoire of molecules (small organic molecules, peptides, polypeptides, etc.) can be screened for those that interact with a repertoire of modified recombinant cell surface receptors (e.g., a receptor with a variable cassette inserted in a region instrumental in ligand binding or activation) by creating an array in which each member of the repertoire of molecules intersects with each member of the repertoire of receptors. Subsequent detection of receptor activation or inhibition in the cells indicates which of the molecules affected the activity of which modified receptor. The process permits both the identification of new modulators of the receptor or other protein, and the rapid identification of structure/function relationships. The method can also be adapted to use higher eukaryotic cells for the expression of both repertoires being analysed for interaction. This would be accomplished, for example, by expressing both repertoires as cell surface molecules, or for example, by expressing one repertoire as a secreted protein and the other as a cell surface protein. Upon contact or close juxtaposition of the cells expressing the respective repertoires, productive interaction of members of each repertoire with members of the other can be detected. One skilled in the art can readily generate higher eukaryotic cells expressing a given repertoire of polypeptides.

Methods of detecting interactions will vary with the exact nature of the array generated. For example, methods will vary depending on whether the array uses cells or not. Multiple detection methods which may be used according to the invention to identify interactions between members of the repertoires in the matrices described herein are well known to those of skill in the art, and may readily be applied to detecting interactions between the molecules described herein. Non-limiting examples of detection methods include:
1. fluorescence resonance energy transfer (FRET; see, for example, Lakowicz, 1986, Principles of Fluorescence Spectroscopy, Plenum, NY; Stryer, 1978, *Ann. Rev. Biochem*, 47: 819-846; and the Molecular Probes catalogue, 6^{th} Ed., Molecular Probes, Eugene, OR);
2. fluorescence quenching (Lakowicz, 1986, Principles of Fluorescence Spectroscopy, Plenum, NY);
3. reporter expression (e.g., luciferase, GST, chloramphenicol acetyltransferase, β-galactosidase, antibiotic resistance; see, for example, Sambrook et al., 1989 Molecular Cloning: A Laboratory Manual 2^{nd} Ed. Cold Spring Harbor Laboratory Press);
4. rescue from auxotrophy (Guthrie and Fink, 1991, Methods in Enzymology, Academic Press);
5. signalling events, such as changes in second messenger levels, GDP for GTP exchange, kinase activation or phosphorylation, phosphatase activation or dephosphorylation, proteolysis or altered ion permeability (Many protocols and reagents for detecting signalling events may be obtained from Calbiochem, La Jolla, CA; and Molecular Probes, Eugene, OR);
6. enzymatic reactions (which can be detected using, for example fluorogenic or chromogenic substrates, Molecular Probes, Eugene, OR);
7. methylation (see, for example Herman et al., 1996, *Proc. Natl. Acad. Sci. USA* 93: 9821-9826; Hibi et al., 2001, *Clin. Cancer Res.* 7: 3135-3138);
8. nucleic acid cleavage (for example, invasive cleavage as described in Wilkins et al., 2001, *Nucleic Acids Res.* 29: E77);
9. glycosylation (methods taught in, among others: Beeley, 1985 Laboratory Techniques in Biochemistry and Molecular Biology, Elsevier; Bouchez-Mahiout et al., 1999, *Electrophysiology,* 20: 1412; Doerner et al., 1990, *Annal. Biochem.* 187: 147; methods and reagents may be obtained from Molecular Probes, Eugene, OR);
10. proteolysis (Demartis et al., 1999, *J. Mol. Biol*. 286:617-633); and
11. infection (e.g., with a virus or phage; see, for example Sambrook et al., 1989 Molecular Cloning: A Laboratory Manual 2^{nd} Ed. Cold Spring Harbor Laboratory Press).

Each of these approaches or read-outs for the detection of interactions is known in the art such that one of ordinary skill can employ them in the methods of the invention without the need for undue experimentation.

Given that interactions which may be screened according to the present invention may involve polypeptides that have been derived, directly or indirectly, by expression of nucleic acid sequences, it is highly advantageous that the nucleic acids themselves are arranged in lines, channels or tubes according to the invention and expressed to produce their corresponding polypeptides. In this way, intersecting polypeptides from each of the two repertoires will be expressed together. This can assist their association, particularly when the association of the two repertoire members depends on co-operative folding, for example, as in the case of antibodies. In addition, information regarding the interactions of members of the repertoires will be spatially linked to the genetic information which encodes them. This genetic information can be determined by calculating the co-ordinates of the interaction and isolating the corresponding nucleotide sequence data from any point on its line, channel or tube or by isolating the nucleotide sequence data from the intersection itself. In one embodiment, the polypeptides are expressed *in vitro* by arranging repertoires of cells which contain nucleic acid sequences encoding the polypeptides of interest, and inducing the expression of the polypeptide. Methods for the expression of polypeptide in cellular systems are well known in the art (see, for example, Oh et al., 1999, *Protein Expr. Puriff.* 17: 428-434; Olson et al. 1998, *Protein Expr. Puriff.* 14: 160-166). In an alternate embodiment, one repertoire comprising a plurality of cells which contain a nucleic acid sequence encoding the polypeptide of interest, and a second repertoire comprising a plurality of nucleic acid molecules incorporated into an expression vector may be arrayed on the same matrix. Using methods known to those of skill in the art, the cellular repertoire may be transfected with the nucleic acid repertoire and induced to express the transfected nucleic acid to generate a polypeptide of interest (Ziauddin and Sabbitini, 2001 *Nature,* 411: 107-110; WO 01/20015).

### Use of molecules selected according to the invention

Molecules selected according to the method of the present invention can be employed in substantially any process. Where the molecules are polypeptides, they can be used in any process which involve binding or catalysis , including *in vivo* therapeutic and prophylactic applications, *in vitro* and *in vivo* diagnostic applications, *in vitro* assay and reagent applications, and the like. For example, antibody molecules can be used in antibody based assay techniques, such as ELISA techniques, Western blotting, immunohistochemistry, affinity chromatography and the like, according to methods known to those skilled in the art (see, for example Sambrook et al., 1989 Molecular Cloning: A Laboratory Manual 2^{nd} Ed. Cold Spring Harbor Laboratory Press).

As alluded to above, the molecules selected according to the invention are of use in diagnostic, prophylactic and therapeutic procedures. For example, enzyme variants generated and selected by these methods can be assayed for activity, either *in vitro* or *in vivo* using techniques well known in the art, by which they are incubated with candidate substrate molecules and the conversion of substrate to product is analysed. Selected cell-surface receptors or adhesion molecules might be expressed in cultured cells which are then tested for their ability to respond to biochemical stimuli or for their affinity with other cell types that express cell-surface molecules to which the undiversified adhesion molecule would be expected to bind, respectively.

Therapeutic and prophylactic uses of proteins prepared according to the invention involve the administration of polypeptides selected according to the invention to a recipient mammal, such as a human. Of particular use in this regard are antibodies, other receptors (including, but not limited to T-cell receptors) or binding protein thereof.

Substantially pure molecules of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected polypeptides can be used diagnostically or therapeutically (including extracorporeally) or in developing and performing assay procedures, immunofluorescent staining and the like (Lefkovite and Pernis, (1979 and 1981) Immunological Methods, Volumes I and II, Academic Press, NY).

The selected antibodies or binding proteins thereof of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight *et al*. (1978) *J. Exp. Med*., **147:** 1653; Reinersten *et al*. (1978) *New Eng. J. Med.,* **299:** 515). Myasthenia Gravis (MG) is tested in SJL/J female mice by inducing the disease with soluble AchR protein from another species (Lindstrom *et al*. (1988) *Adv. Immunol*., **42:** 233). Arthritis is induced in a susceptible strain of mice by injection of Type II collagen (Stuart *et al.* (1984) *Ann. Rev. Immunol*., **42:** 233). A model by which adjuvant arthritis is induced in susceptible rats by injection of mycobacterial heat shock protein has been described (Van Eden *et al.* (1988) *Nature,* **331:** 171). Thyroiditis is induced in mice by administration of thyroglobulin as described (Maron *et al.* (1980) *J. Exp. Med*., **152**: 1115). Insulin dependent diabetes mellitus (IDDM) occurs naturally or can be induced in certain strains of mice such as those described by Kanasawa *et al*. (1984) *Diabetologia,* **27:** 113. EAE in mouse and rat serves as a model for MS in human. In this model, the demyelinating disease is induced by administration of myelin basic protein (see Paterson (1986) *Textbook of Immunopathology,* Mischer *et al.,* eds., Grune and Stratton, New York, pp. 179-213; McFarlin *et al.* (1973) *Science,* **179:** 478: and Satoh *et al*. (1987) *J. Immunol.,* **138**:179).

The selected antibodies, receptors (including, but not limited to T-cell receptors) or binding proteins thereof of the present invention can also be used in combination with other antibodies, particularly monoclonal antibodies (MAbs) reactive with other markers on human cells responsible for the diseases. For example, suitable T-cell markers can include those grouped into the so-called "Clusters of Differentiation," as named by the First International Leukocyte Differentiation Workshop (Bernhard *et al*. (1984) *Leukocyte Typing,* Springer Verlag, NY).

Generally, the present selected antibodies, receptors or binding proteins will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, can be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, can also be present (Mack (1982) *Remington's Pharmaceutical Sciences,* 16th Edition).

The selected polypeptides of the present invention can be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected polypeptides according to the present invention having different specificities, such as polypeptides selected using different target ligands, whether or not they are pooled prior to administration.

The route of administration of pharmaceutical compositions according to the invention can be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The selected polypeptides of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present selected polypeptides or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected antibody, receptor (e.g. a T-cell receptor) or binding protein thereof *per* kilogram of body weight, with doses of 0.05 to 2.0 mg/kg/dose being more commonly used. For prophylactic applications, compositions containing the present selected polypeptides or cocktails thereof can also be administered in similar or slightly lower dosages.

A composition containing a selected polypeptide according to the present invention can be utilised in prophylactic and therapeutic settings to aid in the alteration, inactivation, killing or removal of a select target cell population in a mammal. In addition, the selected repertoires of polypeptides described herein can be used extracorporeally or *in vitro* selectively to kill, deplete or otherwise effectively remove a target cell population from a heterogeneous collection of cells. Blood from a mammal can be combined extracorporeally with the selected antibodies, cell-surface receptors or binding proteins thereof whereby the undesired cells are killed or otherwise removed from the blood for return to the mammal in accordance with standard techniques.

The invention is further described, for the purpose of illustration only, in the following examples.

### Example 1. Matrix Screening of a scFv Repertoire

The two filter capture system used as part of the present example is based upon that described in our co-pending international patent application entitled "Array Screening Method" (Patent Application Number: PCT/GB00/04638). Bacteria are grown in lines on one filter and the scFvs thereby produced are captured on a second filter that has lines of antigen bound to the nitrocellulose, which are oriented at 90° from those lines of scFv on the first filter (see Figure 1). At intersections where scFv interacts with antigen, the scFv is captured if the antigen and scFv bind. In this example, detection of bound scFv is performed with superantigen Protein L conjugated to HRP.

### Methods

### Antigen library

The antigens are from human expression library hEX1, prepared from foetal brain poly(A)+ RNA by oligo(dT)-priming (Büssow et al 1998). cDNAs are cloned directionally in a modified pQE-30 vector (Qiagen).

### Antigen filters

Antigen clones were grown overnight in liquid culture (2xTY containing 100 µg/ml Amp, and 1 % glucose) at 37 °C. For manual line drawing, liquid cultures were transferred to a pre-wetted PVDF filter (soak in ethanol, rinse in PBS and dip in 2xTY) by drawing along the filter against a metal ruler with a p10 filter tip (Art) not mounted on a pipette. Thus, the capillary action of the tip was used for delivery of the liquid onto the surface of the membrane. Each clone was drawn onto the filter 6 mm from the previous one. For automated line drawing, liquid cultures were transferred to a PVDF filter using the robotic head depicted in Fig 3 attached to a Kaybee Systems picker/gridder system. Each clone was drawn onto the filter 4.5mm mm from the previous one at a speed of 25 mm/s.

The antigen filters were then grown overnight on TYE agar plates containing 100 µg/ml Amp, 1 % glucose at 30 °C. The filter was then transferred to another TYE agar plate containing 100 µg/ml Amp, 1mM IPTG for 3h at 37 °C for induction of the clones. Antigen filters were removed from the plate and denatured on pre-soaked blotting paper containing 0.5M NaOH, 1.5 M NaCl for 10 min, neutralised for 2 x 5 min in 1M Tris-HCl, pH7.5, 1.5M NaCl and incubated for 15 min in 2 x SSC. Filters were dried, soaked briefly in ethanol and then blocked in 4 % Marvel PBS, rinsed in PBS and dipped in 2xTY.

### ScFv library

The scFvs are from a library based on a single human framework for V_{H} (V3-23/DP-47 and J_{H}4b) and V_{K} (O12/O2/DPK9 and J_{K}1), with side chain diversity (NNK or DVT encoded) incorporated at positions in the antigen binding site that make contacts to antigen in known structures and are highly diverse in the mature repertoire. The fold that is used is frequently expressed *in vivo*, and binds to the generic ligands Protein L and A, which facilitate capture or detection of the scFvs but do not interfere with antigen binding. The scFv clones have been pre-screened in scFv format for binding to Protein A and Protein L to ensure that they were functional.

### ScFv filter

Antibody clones were grown overnight in liquid culture (2xTY containing 100 µg/ml ampicillin and 1% glucose) at 37°C. Liquid cultures were then transferred to a pre-blocked nitrocellulose filter (4% skimmed milk powder in PBS for 1 hour at room temperature (RT), rinse in PBS and dip in 2xTY). Manual and robotic transfer of antibodies to the filter was performed as for the antigen cultures, except that the density of scFvs lines created by robotic transfer was one every 1.125 mm.

ScFv filters were then grown on TYE agar plates containing 100 µg/ml Amp, 1 % glucose at 37 °C. After overnight growth the antigen filter was placed onto a fresh TYE agar plate 100 µg/ml Amp, 1mM IPTG, dried, and then the scFv filter was placed on top of this. The plate with the two filters was then incubated for 3h at 30 °C for induction of the scFvs.

### Probing of filters

The top (scFv) filter was discarded and the second (antigen) filter was washed 3 x with PBS/0.05% Tween (PBST) and blocked with 4% MPBS for 30 min at RT. The filters were washed 3x with PBST and then incubated with a Protein L HRP conjugate (Actigen, 1/2000) in 4% MPBS for 1 hr at RT. Filters were then washed a further three times with PBST and developed with ECL reagent. All incubations were performed in 50 ml of buffer on a gently agitating shaker.

### Results

As a model system, we performed a manual matrix screen of 21 antigens against 16 scFvs, resulting in 336 interactions being tested using only 37 dispensing events. Included in the scFv repertoire were four scFvs that had been selected against ubiquitin by phage selection (Ub1b1, Ub1a1, R13 and R14). Included in the antigen repertoire were two clones known to be ubiquitin (Q and T) sand five other clones (A, P, R, S and U) that had been identified in a primary screen as probably binding an anti ubiquitin scFv. As can be seen (Fig 2), each of the four anti ubiquitin scFvs bound the two known ubiquitin clones and each of the five potential ubiquitin clones. However, it can be seen that scFv Ub1b1 and scFv R14 are highly cross reactive. Also included in the model array were 14 antigen clones identified in a primary antigen array screen as possible binders to twelve scFv clones C2 to H11. As can be seen from the matrix (Figure 2), antigen M (a protein of unknown function) binds specifically to scFv D12. Also antigen E, (a DNA binding protein) binds specifically to scFv H11. This demonstrates the utility of the matrix screen in confirming interactions originally identified in an antigen array screen.

### Example 2. Robotic creation of a matrix of intersecting antibody/antigen lines.

In this example, we moved to a higher density matrix screen, using a robotic head (Fig 3a - design. Fig 3b - photograph) to draw the lines. In this system double lines of 12 antigens (horizontally) are screened against 192 scFvs (vertically). Thus, 2304 different potential interactions were tested each twice over using only 216 dispensing events. Again many different interactions are observed at the intersections of the lines, particularly against three antigens (two of which are the same). Robotic platforms are well-known in the art, and machines are available from companies such as Genetix (Hampshire, UK), Genetic MicroSystems (Wouburn, MA) and BioRobotics (Wouburn, MA) which are capable of arraying at high speed with great accuracy over small or large surfaces. Such robotic applications may be utilized by one of skill in the art to generate the repertoire matrices of the present invention by following the manufacturers instructions and protocols.

### Example 3. Robotic creation of a matrix of intersecting antibody/antigen lines using a Western blot as one of the dimensions

In this example, a repertoire of scFvs was intersected with a repertoire of size-separated proteins. Double lines of 48 recombinant scFvs were intersected with single lines of size-separated proteins from Bovine Mitochondrial Complex I. One scFv IA111 that was specific for subunit 51 was detected (Figure 10). This was the first demonstration that an antibody-antigen matrix array could be created, using a Western blot of size-separated proteins as the antigen dimension.

### Example 4. Creation and screening of a two-chain antibody repertoire according to the present invention

In this example, a two-chain antibody repertoire is created and screened according to the present invention. The two filter capture system used as part of the present example is based upon that described in our co-pending international patent application entitled "Array Screening Method", (Patent Application Number: PCT/GB00/04638). Previously, it has been shown that antibody heavy and light chains can associate in solution to form Fv fragments that have an active antigen-binding site and such techniques are well known in the art. In order to check whether the non-covalent association of the particular heavy and light chain was of sufficient strength for such association to occur on an array, we split the heavy and light chain of a phage selected anti-BSA scFv (13cg2). As we were unsure how strong the association between heavy and light chains would be, we cloned the 13cg2 heavy and light chains separately into three recombinant fragment formats; heavy or light chain alone (single domains); scFv (with a 15 amino acid linker between heavy and light chain) and diabody (with a zero amino acid linker between heavy and light chain). The latter two formats were constructed with either light or heavy chain 13cg2 diversity, with the non-diversified chain in each case being a dummy heavy or light chain. (The dummy chain has a single but unknown antigen-binding specificity.) Testing of the various formats on the array, using BSA as the antigen, indicates that the diabody formats provide the most stable association on the filter surface.

Bacteria expressing either a Bovine Serum Albumin (BSA) binding heavy chain (1), a BSA binding light chain (2), non binding heavy and light chains (3) or BSA binding heavy and light chains (4), all in the diabody format described above, were either mixed and grown as spots (Figure 5a) or drawn as horizontal and vertical lines and then grown (Figure 5b). In both cases, after overnight growth the filters harbouring the grown bacteria were laid on top of a second filter coated with BSA and then induced for protein expression. Only in those cases where a binding heavy chain is co-expressed with a binding light chain is a positive signal observed (i.e. 1 and 2 together or any combination involving 4. The lack of a signal for 1 down with 2 across is probably due to a bubble being present between the filters during induction). The drawing of lines dramatically reduces the number of dispensing events (in this case from 32 to 8).

### Example 5. Creation of a high-density array of 147,456 antibodies

In this example, the density of the heavy and light chain lines was increased allowing higher density antibody arrays to be created and screened. In addition a novel, three-filter system was used so that the scFv lines can be drawn on different filters (Figure 11) Briefly, lines of bacteria containing genes for recombinant antibody heavy chains (V_{H}) are grown on one membrane. Lines of bacteria containing genes for recombinant antibody light chains (V_{L}) are grown on a second membrane (a). The membranes are stacked one on top of the other (with their lines of bacteria at right angles) and placed on a third membrane coated in target antigen. Bacteria are then induced to express their antibody fragments (see, for example, Oh et al., 1999, *Protein Expr. Puriff.* 17: 428-434; Olson et al. 1998, *Protein Expr. Puriff.* 14: 160-166) and the V_{H} and V_{L} diffuse through the top two membranes to the antigen coated membrane (b). At intersections between lines, V_{H} and V_{L} associate and form a complete, active antigen-binding site. Those V_{H} and V_{L} combinations that do not bind antigen are washed away. Any V_{H} and V_{L} combinations that bind to the target antigen are captured on the antigen membrane and detected with Protein L-HRP, as described in Example 1.

Thus, 24 anti-BSA heavy chains and 48 anti-BSA light chains were drawn perpendicular to the x and y axes to create 1152 pairings screened against BSA (Figure 6). In an even higher density format 384 unselected heavy chains and 384 unselected light chains were drawn perpendicular to the x and y axes and screened against BSA coated onto a nitrocellulose filter (147,456 combinations). A single specific heavy and light chain pairing was isolated which was subsequently confirmed as binding to nitrocellulose (Figure 7). If the screen were to be increased to cover 1000 heavy chains versus 1000 light chains (1 million different antibodies) the number of dispensing events would be reduced from 2 million to 2 thousand by using the method according to the present invention.

### Example 6. Creation of a matrix to detect protein-protein interactions

This example shows a potentially general strategy for the investigation of interactions among polypeptides (and in particular extracellular proteins) that obviates the need for purified target protein. It is based on the periplasmic expression of receptors fused to a V kappa domain (FLAG tag or gene 3 display also works) and ligands fused to a dimeric fusion protein glutathione-S-transferase (GST)). This allows sensitive detection of interacting pairs by capture of the dimeric GST fusion with an anti-GST antibody and detection of the scFv or V kappa fusion protein with Protein L conjugate using a two-filter based screening method (De Wildt *et al*. (2000) *Nature Biotech*., 9:989). We demonstrate the utility of this method for the rapamycin-dependent interaction between FRB/FKBP-12 (Brown *et al,* (1994) *Nature,* 369:756). FKBP-12 is fused to GST and FRAP is fused to a V kappa domain. (FLAG tag also works). Cognate pairs can be detected only in the presence of rapamycin in soluble ELISA as well as when using line drawing (Figure 12) using the two-filter based screening method.

### Example 7. Three-dimensional screening

A schematic for three-dimensional screening according to the invention is shown (Figure 8). Here, members of the repertoires are arranged in planes in the x, y and z axes and the interactions occur at the various vertices of the intersecting planes.
As a proof of concept, to show that an interaction between three elements can be detected at a vertex, an anti-BSA heavy chain is deposited on one plane, an anti-BSA light chain is deposited on a second plane, and BSA is deposited in the third plane. An interaction at their vertex is detected only when all three are present (Figure 9).

In this example, a two-chain antibody is recreated and screened for binding to BSA according to the present invention. The three filter capture system used as part of the present example is based upon the two filter capture system described in our co-pending UK patent application entitled "Array Screening Method", (UK Patent Application Number: 9928787.2). Bacteria expressing a Bovine Serum Albumin (BSA) binding heavy chain in the diabody format described above, are grown as a lawn on a nitrocellulose membrane resting on an agar plate. At the same time, bacteria expressing a Bovine Serum Albumin (BSA) binding light chain in the diabody format described above, are grown as a lawn on a separate nitrocellulose membrane resting on an agar plate. After overnight growth, the filters harbouring the grown bacteria are placed on separate agar plates containing IPTG induction media and rectangular pieces of the IPTG-agar/nitrocellulose/bacteria sandwich are excised and placed at right angles to each other so that the junction between the two pieces of nitrocellulose comes into contact, at a vertex, with a third filter coated with BSA (Figure 9a). Pieces of membrane and agar are held in place by a rig that has three planes coming together to form a right-angle. The whole ensemble is then incubated at 30 °C to induce bacterial expression of recombinant proteins. Only at a vertex where a binding heavy chain is co-expressed with a binding light chain in the presence of the target antigen (Fig 9b) is a positive signal observed. Where a dummy V_{H} is used in place of the binding V_{H}, no signal is detected (Figure 9c). Where a filter coated with Marvel is used in place of a filter coated in BSA no signal is detected (Figure 9d).

### Example 9 Creation and screening of a combinatorial chemical library

Combinatorial synthesis is described in International Patent Application WO 94/08051, published Apr. 14, 1994; and Lowe, Gordon, "Combinatorial Chemistry," Chem. Soc. Rev., 1995, pp. 309-317.
Creation of chemical libraries requires parallel synthesis of a library of varied complex molecules which have a range of different functional groups. One approach to creating such a repertoire is to synthesise a core structure with multiple sites to which functional groups can be added. Then, varying combinations of functional groups are added onto the core molecule in a series of organic synthesis steps. For efficiency, it is necessary to minimise the number of organic synthesis steps to create the library. One way to achieve this is a split and pool approach, in which a collection of core molecules, each of which is modified in a different way are simultaneously subjected to a single organic synthesis step, adding the same functional group to all the different molecules.

In this example, a combinatorial chemical library is created and screened using a matrix array. An initial organic synthesis step is conducted in the conventional manner (see, for example WO 94/08051; Lowe, 1995, *Chem. Soc. Rev*., pp. 309-317), to create a library of molecules all containing the core domain and one functional side group. These molecules are dispensed onto an array as lines either by hand or using a robotic dispensing apparatus as described above, and an unmodified site is activated in all members of the repertoire. A second repertoire of lines, each representing an alternative side group is then dispensed onto the array in activated form. The second repertoire of lines is juxtaposed to the first. At each intersection a unique combination of new side chain and modified core domain is brought together and reacts to form a unique molecule.

A larger library can be created by increasing the complexity of the first and second repertoires. For example, the core domain repertoire can be expanded by conducting multiple synthesis steps before arraying, so that each line represents a single molecular species in which the core structure has one or more than one functional groups already added. In this case, only the last synthesis step will then be conducted on the array. Alternatively, an extremely large repertoire of activated functional groups can be used, so that a wide variety of functional moieties are added onto each modified core domain.

Core domains, reactive functionalities, and side groups, useful in this embodiment of the present invention are readily available from commercial vendors, such as Sigma (St. Louis, MO), and/or are known to those of skill in the art. Examples of chemical units which may function as core groups, reactive functionalities, or side chains include both naturally-occurring and synthetic units, such as nucleophiles, electrophiles, dienes, alkylating or acylating agents, diamines, nucleotides, amino acids, sugars, lipids, or derivatives thereof, organic monomers, synthons, NH₂, SH, OH, CN, halogens, methacrylate, quaternary amine salts, carboxylic acids and salts, phosphonates, succinic anhydride, 2-carbomethoxyaziridine, dihydroimidazole, thiocyanato, isocyanato, isopropeno, 2,3-epoxypropoxy, epoxy-alkyl, and combinations thereof. Reactions which may be employed by those of skill in the art to generate the combinatorial chemical libraries useful in the present invention include ionization, combustion, polymerization, hydrolysis, condensation, enolization, saponification, rearangement, alkylation, acylation, nitration, halogenation, oxidation, reduction, substitution, elimination, addition, and the like. Such reactions, using chemical units available to those of skill in the art, can produce non-oligomers, oligomers, or combinations thereof, including a wide variety of organic molecules, such as heterocyclics, aromatics, alicyclics, aliphatics and combinations thereof, comprising steroids, antibiotics, enzyme inhibitors, ligands, hormones, drugs, alkaloids, opioids, terpenes, porphyrins, toxins, catalysts, as well as combinations thereof. Oligomers include oligopeptides, oligonucleotides, oligosaccharides, polylipids, polyesters, polyamides, polyurethanes, polyureas, polyethers, poly (phosphorus derivatives) e.g. phosphates, phosphonates, phosphoramides, phosphonamides, phosphites, phosphinamides, etc., poly (sulfur derivatives) e.g. sulfones, sulfonates, sulfites, sulfonamides, sulfenamides, etc., where for the phosphorous and sulfur derivatives the indicated heteroatom for the most part will be bonded to C, H, N, O or S, and combinations thereof.

The specific techniques, including chemical units, reactive functionalities, side groups, and reaction conditions are well known to those of skill in the art and are described, for example, in WO 94/08051 and Lowe, 1995, *Chem. Soc. Rev.,* pp. 309-317.

The most efficient way to use the matrix approach however, is to iteratively use matrices to create a repertoire. In this way each member of the repertoire created on the first matrix array is used to draw a line on the second matrix. A new active site is unmasked in all the repertoire members and then a third functional group is added by drawing lines. This new repertoire, in which each member is modified at three sites on the core domain is then used to draw another matrix and so on. This iterative strategy is continued until all the sites on the core molecule suitable for fuctionalisation have been modified.

Whichever matrix strategy is used to create the combinatorial chemical library, molecules on the final array are then screened for possible activity. One way to achieve this is to screen the array against a lawn of adhesive human cells to identify any novel molecules with cytotoxic activity. For example, bacterial cells, such as *E. coli* may be grown to confluence on suitable medium as described in Sambrook et al. (*supra*). In one embodiment, the combinatorial chemical matrix is generated as described above on a nylon membrane or other suitable substrate. The membrane may then be placed on the bacterial culture, such that the chemicals present at each intersection of the matrix are juxtaposed to the bacterial cells. The culture/matrix may be incubated as directed for *E. coli* cultures for 2-48 hours. Subsequently, the membrane is removed and the bacterial culture may be evaluated for, for example, cell death (as evidenced by plaques, for example) in defined regions corresponding to specific points of intersection of the repertoires on the membrane. Identification of cellular mortality in regions of the culture may then be indicative of the antimicrobial properties of the combinatorially derived chemical at the corresponding position on the matrix.

Combinatorial chemical libraries constructed in this manner may also be screened for possible activity against any of the molecular interactions described herein.

### Example 10 Creation and storage of a combinatorial chemical library

In this example, a combinatorial chemical library is created and stored using matrix array technology. A matrix array is synthesised as described in example 9 and used to store the repertoire of molecules. The stored array can be used for future screening for possible activity, either 'on array' as described above, or by extracting the compounds and screening them in another location. For example, if the matrix is produced on a nylon membrane or filter, the points of intersection of the repertoires may be physically removed from the matrix (e.g., by cutting out the portion of membrane or filter). The portion of membrane or filter may then be extracted in an appropriate solvent to recover the combinatorial adduct. For example, depending on the polarity of the combinatorial adduct, one of skill in the art would recognize the need to use an appropriately polar or non-polar solvent (e.g., water, ethanol, chloroform, benzene, and the like). Methods of extracting chemicals from porous substrates are known in the art. The matrix storage array can also be used as a resource from which to retrieve samples of an individual potential lead compound already shown to have desired activity.

### Example 11 Screening of proteases against peptide sequences or protein substrates

In this example, a library of proteases is screened against a library of peptide sequences. A library of peptides is synthesised on an array using the SPOT synthesis technique (Frank, R. (1992). Spot synthesis: An easy technique for the postionally addressable, parallel chemical synthesis on a membrane support. *Tetrahedron* 48, 9217-9232.) except that peptides are synthesised in lines rather than spots. Each peptide has one or more constant regions (the same amino acid sequence throughout the library) and one or more variable regions representing possible protease cleavage sites. The peptides are labelled during peptide synthesis with a fluorescence dye at the amino-terminus. (Duan Y and Laursen RA (1994): Protease substrate specificity mapping using membrane-bound peptides. *Anal Biochem*, 216: 431-438). Meanwhile, a library of putative proteases is synthesised by active site directed mutagenesis of DNA coding for a known protease, followed by transformation of *E. coli* with library DNA and bacterial expression of the recombinant protein (see Babe LM, Linnevers CJ, Schmidt BF (2000) Production of active mammalian and viral proteases in bacterial expression systems. *Biotechnol Genet Eng Rev* 17:213-52.) Each protease is then separately purified and resuspended in buffer. The library of proteases is then dispensed onto the matrix array as lines which are at right angles to the library of peptides, so that each peptide intersects with each protease. Time is allowed to lapse for any active proteases to cleave peptide, before adding a protease inhibitor to stop enzymatic reaction. The array is then washed, removing both inactivated proteases and the carboxy terminus of any cleaved peptides. The array is then probed with two colours of detection reagent, one that binds the tag at the carboxy terminus of the peptide and one that binds the tag at the amino terminus. Spots of cleaved peptide (corresponding to the positions where a line of active enzyme intersects with a line of peptide that is a suitable substrate for cleavage) are detected by an excess of carboxy terminal signal over amino terminus signal.

A variation on this experiment is to use a library of proteins as potential substrates for the library of putative proteases. Again a dual labelling approach is used to capture the protein at one position and detect it at another. The positions for labelling could again be the carboxy and amino terminus or could be two epitopes between which a putative enzymatic cleavage site was thought to lie.

### Example 12 Screening of enzymes against substrates

This example demonstrates a method by which a repertoire of enzymes is screened against a repertoire of potential substrate molecules. The methods of the present invention may be used to screen any enzyme known to those of skill in the art, including, but not limited to kinases, phosphatases, gastric enzymes, proteases, and the like. For example, a repertoire of protein kinases is screened against a repertoire of possible protein substrates, to determine which kinases phosphorylate which substrates. A library of human kinases is constructed using protocols for genome-wide kinase identification and cloning similar to those used by Zhu et al. (2000) *Nature Genetics* 26, 283-289. Protein expression is in recombinant cell lines, which are of bacterial, yeast, mammalian or other origin and may be using a vector system compatible with multiple cell types such as that described by Leuking *et al*. (2000); *Protein expression and purification* 20, 372-378. The kinases are purified and resuspended in buffer. Meanwhile a library of human proteins is constructed and expressed in recombinant cell lines, which may be bacterial, mammalian or of another origin. Protein expression may be using a vector system compatible with multiple cell lines such as that described by Leuking *et al*. (2000); *Protein expression and purification* 20, 372-378. The proteins are purified and resuspended in buffer. The putative protein substrates are deposited onto BSA-NHS glass slides as lines and then the kinases are deposited as juxtaposed lines onto the same slide. The slide is incubated in the presence of [γ-³³P] adensoine phosphate (as described by MacBeath and Schreiber (2000); *Science* 289,1760-1763). To detect isotopically labelled phosphorylated proteins, slides are dipped into a photographic emulsion and developed manually, resulting in the deposition of silver grains directly onto the glass surface. The slides are then visualised using a light microscope. Any intersections where an active kinase contacts a suitable substrate protein result in the deposition of silver grains. (As a positive control, casein kinase II is included in the kinase repertoire and its substrate, protein phosphatase inhibitor 2 is included in the substrate repertoire.)

### Example 13 Screening of human proteins to detect protein-protein interactions

In this example, a repertoire of human proteins is screened against a second repertoire of human proteins to search for binding interactions between the two. This experiment could be conducted with a few human proteins or, given a suitable set of cloned proteins, with every human protein.

Briefly, human proteins are expressed in recombinant cell lines, which may be bacterial, mammalian or of another origin, using methods known to those of skill in the art (Oh et al., 1999, *Protein Expr. Puriff*. 17: 428-434; Olson et al. 1998, *Protein Expr. Puriff*. 14: 160-166). Protein expression may be using a vector system compatible with multiple cell lines such as that described by Leuking *et al.* (2000); *Protein expression and purification* **20,** 372-378. Two repertoires of human proteins are constructed, (which may each contain all or some of the same proteins). The proteins in the first repertoire are labelled with a tag such as GST and the proteins in the second repertoire are labelled with a tag such as flag. The use of tags to label proteins is well known in the art. Tags, useful in the present invention include, but are not limited to His, Myc, GST, Flag, and VSV (Hopp et al., 1988, *Biotechnology* 6: 1205-1210; Kreis, 1986, *EMBO J*. 5: 931-941; Kaelin et al., 1992 *Cell* 70: 351). Tagged proteins from each repertoire are affinity purified by means of the tag.

Purified proteins from the first repertoire are drawn onto the array as lines and captured by means of the GST tag, followed by washing steps. Methods and reagents for the purification and capture of proteins by means of tags are known in the art and may be obtained from, for example Sigma (St. Louis, MO), Boehringer Mannheim (Indianapolis, IN), and APB Biotech (Piscataway, NJ). Proteins from the second repertoire (labelled with flag) are then dispensed as lines at right angles to those from the first repertoire so that every protein from the second repertoire intersects with every protein from the first repertoire. Any flag labelled proteins that have sufficient affinity for GST labelled proteins are captured and then the array is washed. Any flag labelled proteins not bound to a partner are washed away. Remaining flag labelled proteins are then detected with an anti-tag antibody or other suitable affinity ligand, labelled with Cy 3 or another suitable detectable marker.

At intersections where a protein-protein interaction occurs there will be a Cy 3 signal. One interesting aspect of this experiment is that any protein that interacts with the tag of proteins in the target repertoire rather than the target proteins themselves will be easily be identified as a false positive as it will be represented by a line (or series of spots) of Cy 3 signal.

### Example 14 Screening of different cellular fractions against different antibodies

In this example, lines of monoclonal recombinant antibodies from a repertoire isolated by phage display (see, for example U.S. Pat. Nos. 5,702,892; 5,824,520) are dispensed onto an array either using piezoelectric dispensation or capillary action dispensation similar to that described in this document. Antibodies are either captured by a generic ligand such as protein L that has been captured on a derivitised slide or the antibody fragments are directly captured on the derivitised slide. Slide preparation, buffers and protocols for capturing protein L or antibodies are as described by Haab (Haab, B. B., Dunham, M. J. and Brown, P. O. (2001). Protein microarrays for highly parallel detection and quantitation of specific proteins and antibodies in complex solutions. *Genome Biology 2 ,* research 0004.1-0004.13.). The array is then blocked with Marvel or another suitable blocking reagent. Meanwhile, cellular fractions of interest are prepared using techniques known in the art. (Many protocols for preparing cellular fractions can be found in "Cell Biology A Laboratory Handbook. Academic Press 1994 *Editor J. E. Celis* ISBN 0-12-164715-3" Examples of such techniques include 'preparation of nuclei from rat liver' and 'preparation of nuclear envelopes from rat liver nuclei' and 'preparation of ribosomes'.)

Proteins in each of the samples are labelled using Cy-5, then each different protein sample is mixed with an aliquot of a standard reference sample labelled with Cy-3 as described by Haab et al. (Haab, B. B., Dunham, M. J. and Brown, P. O. (2001). Protein microarrays for highly parallel detection and quantitation of specific proteins and antibodies in complex solutions. *Genome Biology 2 ,* research 0004.1-0004.13.) Protein samples are deposited onto the array as lines which intersect with the lines of captured antibodies. Lines of protein may be contained spatially on the array by channels. Dispensation of proteins is either using piezoelectric dispensation or capillary action dispensation similar to that described in this document. Incubation and washing again follow the protocols outlined by Haab et al. Any proteins present in the samples which bind specifically to an antibody on the array are captured at the intersection with that antibody. After washing away unbound proteins from the cellular fractions, bound proteins are detected by means of the label, for example by an Axon laboratories scanner or similar. This technique may be used to analyse differences between the same cellular fraction from different patients, for example. Another possible application is to analyse difference between diseased and non-diseased tissue from the same patient.

Alternatively, two repertoires of different cellular fractions may be generated as described above and arranged in a series of intersecting parallel lines, such that each cellular fraction of one repertoire is juxtaposed to each cellular fraction of the second repertoire. The resulting matrix may thus be screened for interactions between the two repertoires using the detection methods described herein.

### Example 15 screening of different tumour cell lines against different antibodies

In this example, lines of monoclonal recombinant antibody from a repertoire isolated by phage display are screened against cell surface proteins from various adherent tumour cell lines. Adherent tumour cells from culture (stocks available from a source such as the European Collection of Animal Cell Cultures (Wiltshire, UK), the American Type Culture Collection (Manassas, VA) or obtained from primary culture) are dispensed onto an array coated for tissue culture using methods well known in the art. Cells from the different cell lines are dispensed as lines. The lines of cells may or may not be constrained by dividers. Each line of cells is labelled with a unique quantum dot tag (Han M, Gao X, Su JZ and Nie S (2001) Quantum-dot-tagged microbeads for multiplexed optical coding of biomolecules. Nat Biotechnol 19:631-5). Labelling is by a method well known in the art for ubiquitous cell surface labelling such as loading the clatherin pits (A full protocol for loading Clatherin pits may be found in the Molecular Probes catalogue).

After washing away excess ubiquitous label, lines of antibodies, each individually labelled using a unique quantum dot tag (Han M, Gao X, Su JZ and Nie S (2001) Quantum-dot-tagged microbeads for multiplexed optical coding of biomolecules. Nat Biotechnol 19:631-5) are dispensed onto the array as lines at right angles to the lines of cells. Cells are incubated with labelled antibodies using buffers well known in the art for cell surface labelling. After labelling, all the cells on the array are washed several times to remove unbound antibodies prior to resuspending the cells in one vial using tissue culture resuspension techniques well known in the art. Cells are then sorted by flow assisted cell sorting (FACS) using their quantum dot microbead tags. Techniques for flow assisted cell sorting are well known in the art, although the FACS scanner has to be modified to detect the wavelengths of light emitted by the quantum-dot tagged microbeads. Any cells labelled with antibody-specific tags can be sorted in a second dimension by their cell line-specific tag. This allows the operator to identify which cell lines are bound by which antibodies.

### Example 16 Creation of a combinatorial DNA library for transcription and translation

In this example, the technology described by Riechmann and Winter *Proc Natl Acad Sci USA* **97**, 10068-10073) which involved recreating functional domains by ligating the DNA from half a domain with DNA from partial cDNAs is achieved on an array. Two repertoires of double stranded DNA, with complementary sticky ends are dispensed onto an array as lines. DNA is then ligated *in situ* at the intersections between lines. Methods for the generation of sticky ends and DNA ligation may be found in the art, including numerous laboratory texts and manuals such as Sambrook et al. (*supra*). *In vitro* translation mix is used to translate each different piece of ligated DNA *in situ* on the array. An array of chimeric proteins results, with each protein composed of amino acids translated from DNA originating from two different repertoires. These proteins can be tested for functionality using a range of assays, as described above, for qualities such as binding and catalysis. Alternatively, the ligated nucleic acid molecules may be transfected into, for example, bacterial cells using methods known in the art (see for example Ziauddin and Sabbatini, 2001, *Nature* 411: 107-110; WO 01/20015). The proteins may then be expressed in the bacterial cells and screened for functionally as described. Alternatively, the chimeric protein matrix resulting from the above methods may be screened against other repertoires including, protein, antibody, cell, virus, etc. according to the methods described herein.

### Example 17 Screening small molecules against repertoires of the invention

In one embodiment, the present invention provides a method for screening a repertoire comprising a plurality of small molecules against a number of other repertoires including cells, cell fractions, viruses, proteins, nucleic acid, amino acids, antibodies, patient samples, tumor biopsies, and the like. For example, a repertoire comprising a plurality of small molecules may be generated using methods known to those of skill in the art (see, for example DeWitt et al., Proc. Natl. Acad. Sci., USA 90:690, 1993; Bunin et al., Proc. Natl. Acad. Sci., USA 91:4708, 1994). A small molecule, useful in the present invention is a compound which is not itself the product of gene transcription or translation (protein, RNA or DNA). Preferably a "small molecule" is a low molecular weight compound, e.g., less than 7500 amu. Examples of small molecules which may be screened against repertoires according to the invention include, beta-lactam antibiotics, steroids, retinoids, polyketides, organic molecules, for example heterocyclics, aromatics, alicyclics, aliphatics, steroids, antibiotics, enzyme inhibitors, ligands, hormones, drugs, alkaloids, opioids, terpenes, porphyrins, toxins, catalysts, as well as combinations thereof.

A repertoire of small molecules may be deposited on an array as a series of parallel lines disposed at right angles to a second series of parallel lines representing a second repertoire of, for example, different cell types, such that each small molecule intersects with each cell type. The array is allowed to incubate at appropriate conditions for the cell types used. Subsequently the array is washed in a suitable buffer and observed to determine whether an interaction occurred between each of the small molecules and each of the cell types. For example, if the small molecules tested represent candidate antibiotics, the points of intersection of the two repertoires may be screened for cell mortality using indices known in the art, such as viability or cytotoxicity assays available from commercial vendors such as Molecular Probes (Eugene, OR).

One of skill in the art, following the teachings of the present invention would be able to adapt these methods to screen for interactions between small molecules and other repertoires such as virus strains, tumor cells, protein, nucleic acid molecules, and the like.

### Other Embodiments

The foregoing examples demonstrate experiments performed and contemplated by the present inventors in making and carrying out the invention. It is believed that these examples include a disclosure of techniques which serve to both apprise the art of the practice of the invention and to demonstrate its usefulness. It will be appreciated by those of skill in the art that the techniques and embodiments disclosed herein are preferred embodiments only that in general numerous equivalent methods and techniques may be employed to achieve the same result.

All of the references identified hereinabove, are hereby expressly incorporated herein by reference to the extent that they describe, set forth, provide a basis for or enable compositions and/or methods which may be important to the practice of one or more embodiments of the present inventions.

## Claims

1. A method for screening a first repertoire of members against a second repertoire of members to identify members of the first repertoire which interact with members of the second repertoire, comprising :
(a) providing an array of members of the first repertoire juxtaposed with members of the second repertoire which permits interaction of said first repertoire members with said second repertoire members, said array comprising a solid surface, said first repertoire present on a solid surface in a first series of continuous, non-intersecting lines such that each line of said series comprises a member of said first repertoire, and said second repertoire present on a solid surface in a second series of continuous, non-intersecting lines such that each line of said series comprises a member of said second repertoire, such that members of the first repertoire are juxtaposed to members of the second repertoire; and
(b) detecting an interaction between members of the first and second repertoires, thereby identifying a member of the first repertoire that interacts with a member of the second repertoire.

2. The method according to claim 1, wherein each of said continuous, non-intersecting line of each of said first and second series comprises a different member of said first and second repertoires, respectively.

3. The method according to claim 1 wherein said line comprises a channel, and wherein members of said first and second repertoires placed continuously in said channel, and wherein said channel is cut into a solid material such that each channel containing a member of the first repertoire intersects each channel containing a member of the second repertoire.

4. The method according to claim 1, wherein said first and second series of continuous, non-intersecting lines are present on the same solid support, such that each member of said first repertoire is juxtaposed to each member of said second repertoire.

5. The method according to claim 1, comprising the steps of:
(a) applying said first repertoire to a first solid surface in a first series of continuous, non-intersecting lines such that each line of said series comprises a member of said first repertoire, applying said second repertoire to a second solid surface in a second series of continuous, non-intersecting lines such that each line of said series comprises a member of said second repertoire; and;
(b) juxtaposing the first and second supports such that all members of the first repertoire are juxtaposed with all members of the second repertoire; and
(c) detecting an interaction between the members of the first and second repertoires.

6. A method for screening a first repertoire of members against a second repertoire of members to identify members of the first repertoire which interact with members of the second repertoire, comprising:
(a) providing an array of members of the first repertoire juxtaposed with members of the second repertoire which permits interaction of said first repertoire members with said second repertoire members, said array comprising said first repertoire present in the lumen of a first series of non-intersecting tubes, such that each tube of said series comprises a member of said first repertoire, and further comprising said second repertoire present in the lumen of a second series of non-intersecting tubes, such that each tube of said series comprises a member of said second repertoire, such that the lumen of each of said first series of tubes intersects with the lumen of each of said second series of tubes, such that members of said first repertoire are juxtaposed to members of said second repertoire.
(b) detecting an interaction between members of the first and second repertoires, thereby identifying a member of the first repertoire that interacts with a member of the second repertoire.

7. A method of making an array for screening a first repertoire of members against a second repertoire of members to identify members of the first repertoire which interact with members of the second repertoire comprising:
(a) providing at least one solid surface;
(b) depositing on said solid surface a said first repertoire as a series of continuous, non-intersecting lines, such that each of said first series of continuous, non-intersecting lines comprises a member of said first repertoire;
(c) depositing on said solid surface said second repertoire as a series of continuous, non-intersecting lines, such that each of said second series of continuous, non-intersecting lines comprises a member of said second repertoire, and such that each of said second series of lines intersects with each of said first series of lines, and said first repertoire of members is juxtapose to said second repertoire of members.

8. A method for screening first, second, and third repertoires of molecules against each other to identify members of the first, second and third repertoires which interact comprising:
(a) providing an array of members of first, second, and third repertoires juxtaposed to each other which permits interaction of said first, second and third repertoire members, said array comprising three solid surfaces, a member of said first repertoire present on a first solid surface of said array, a member of said second repertoire present on a second solid surface of said array, and a member of said third repertoire present on a third solid surface of said array, such that each of said first, second, and third solid surfaces of said array intersect, such that members of the first, second and third repertoires are juxtaposed; and
(b) detecting an interaction between members of the first, second and third repertoires, thereby identifying members of the first, second and third repertoires that interact.

9. The method of claim 7, wherein said array is a cube.

10. A method for screening first, second and third repertoires of members against each other to identify members of the first, second and third repertoires which interact, comprising :
(a) providing an array of members of first, second and third repertoires, juxtaposed to each other which permits interaction of said first, second and third repertoire members, said array comprising a solid surface, said first repertoire present on a solid surface in a first series of continuous, non-intersecting lines such that each line of said series comprises a member of said first repertoire, said second repertoire present on a solid surface in a second series of continuous, non-intersecting lines such that each line of said series comprises a member of said second repertoire, and said third repertoire present on a solid surface in a series of continuous, non-intersecting lines such that each line of said series comprises a member of said third repertoire, and such that members of said first, second and third repertoire is juxtaposed to other members of said first, second, and third repertoires; and
(b) detecting an interaction between members of the first, second and third repertoires, thereby identifying members of the first, second and third repertoires that interact.

11. A method for creating a combinatorial library of two-chain polypeptides, wherein each member of said library comprises one member of a first repertoire of single chain polypeptides and one member of a second repertoire of single chain polypeptides, which method comprises the step of providing an array of members of the first repertoire juxtaposed with the members of the second repertoire which permits interaction of said first repertoire members and said second repertoire members, said array comprising a solid surface wherein said first and second repertoires of single chain polypeptides are present on a solid surface in a first and second series of continuous, non-intersecting lines, respectively, such that each line of said first series intersects with each line of said second series, such that members of the first repertoire are juxtaposed members of the second repertoire, thereby generating two-chain polypeptides at the intersection of said first and second series, thereby creating a combinatorial library of two-chain polypeptides.

12. A method of screening a combinatorial library of two-chain polypeptides for binding to a target molecule, wherein said library is produced by a method comprising providing an array of members of a first repertoire juxtaposed with the members of a second repertoire which permits interaction of said first repertoire members and said second repertoire members, said array comprising a solid surface wherein said first and second repertoires of single chain polypeptides are present on a solid surface in a first and second series of continuous, non-intersecting lines, respectively, such that each line of said first series intersects with each line of said second series, such that members of the first repertoire are juxtaposed members of the second repertoire, thereby generating two-chain polypeptides at the intersection of said first and second series, thereby creating a combinatorial library of two-chain polypeptides, the method comprising the steps of contacting said combinatorial library with said target molecule, and detecting the interaction between the two chain polypeptide and the target molecule.

13. The screening method according to claim 12, wherein the combinatorial library is screened for interactions with more than one target molecule.

14. A method for creating a combinatorial library of three chain polypeptides wherein each member of said library comprises one member of a first repertoire of single chain polypeptides, one member of a second repertoire of single chain polypeptide, and one member of a third repertoire of single chain polypeptides, which method comprises the step of providing an array of members of first, second, and third repertoires juxtaposed to each other which permits interaction of said first, second and third repertoire members, said array comprising three solid surfaces, a member of said first repertoire present on a first solid surface of said array, a member of said second repertoire present on a second solid surface of said array, and a member of said third repertoire present on a third solid surface of said array, such that each of said first, second, and third solid surfaces of said array intersect, such that members of the first, second and third repertoires are juxtaposed, thereby generating three-chain polypeptides at the intersection of said first, second, and third solid surfaces, thereby creating a combinatorial library of three-chain polypeptides.

15. A method of screening a combinatorial library of three-chain polypeptides for binding to a target molecule, wherein said library is produced by a method comprising providing an array of members of first, second, and third repertoires juxtaposed to each other which permits interaction of said first, second and third repertoire members, said array comprising three solid surfaces, a member of said first repertoire present on a first solid surface of said array, a member of said second repertoire present on a second solid surface of said array, and a member of said third repertoire present on a third solid surface of said array, such that each of said first, second, and third solid surfaces of said array intersect, such that members of the first, second and third repertoires are juxtaposed, thereby generating three-chain polypeptides at the intersection of said first, second, and third solid surfaces, thereby creating a combinatorial library of three-chain polypeptides, the method comprising the steps of contacting said combinatorial library with said target molecule, and detecting the interaction between the three chain polypeptides and the target molecule.

16. The method of claim 14 or 15, wherein said combinatorial library is screened for interactions with more than one target molecule.

17. A method for creating a combinatorial library of three-chain polypeptides, wherein each member of said library comprises one member of a first repertoire of single chain polypeptides, one member of a second repertoire of single chain polypeptides, and one member of a third repertoire of single chain polypeptides, which method comprises the step of providing an array, comprising a solid surface, wherein the first, second, and third repertoires of single chain polypeptides are present on a solid surface in a first, second, and third series of continuous, non-intersecting lines, respectively, such that each line of said first series intersects with each line of said second and third series, each line of said second series intersects with each line of said first and third series, and each line of said third series intersects with said first and second series, such that members of the first, second and third repertoires are juxtaposed to each other, thereby generating three-chain polypeptides at the intersection of said first, second, and third series, thereby creating a combinatorial library of three-chain polypeptides.

18. A method of screening a combinatorial library of three-chain polypeptides for binding to a target molecule, wherein said library is produced by a method comprising providing an array, comprising a solid surface, wherein a first, second, and third repertoire of single chain polypeptides are present on a solid surface in a first, second, and third series of continuous, non-intersecting lines, respectively, such that each line of said first series intersects with each line of said second and third series, each line of said second series intersects with each line of said first and third series, and each line of said third series intersects with said first and second series, such that members of the first, second and third repertoires are juxtaposed to each other, thereby generating three-chain polypeptides at the intersection of said first, second, and third series, thereby creating a combinatorial library of three-chain polypeptides, the method comprising the steps of contacting said combinatorial library with said target molecule, and detecting the interaction between the three chain polypeptides and the target molecule.

19. The screening method according to claim 17 or 18, wherein the combinatorial library is screened for interactions with more than one target molecule.

20. The method according to claim 1, whereby one or both of the first or second repertoires comprises a plurality of nucleic acid molecules which are expressed to produce their corresponding polypeptides *in situ* in the array.

21. The method according to claim 8 or 10, whereby one or all of the first, second and third repertoires comprises a plurality of nucleic acid molecules which are expressed to produce their corresponding polypeptides *in situ* in the array.

22. The method according to claim 20 or 21, wherein the nucleic acid molecules are in the form of expression vectors which encode polypeptide members of the repertoire, operatively linked to control sequences sufficient to direct the transcription of the nucleic acid molecules.

23. The method according to claim 22, wherein the expression vector is a bacteriophage.

24. The method according to claim 22, wherein the expression vector is a plasmid.

25. The method according to claim 22, wherein the expression vector is a linear nucleic acid molecule.

26. The method according to claim 22, wherein the nucleic acids are contained and expressed within cells.

27. The method according to claim 26, wherein the cells are selected from the group consisting of prokaryotic and eukaryotic cells.

28. The method according to claim 1 or 10, wherein the members of at least one repertoire are arranged in said series of continuous, non-intersecting lines using robotic means.

29. A method for screening a first repertoire of members against a second repertoire of members to identify members of the first repertoire which interact with members of the second repertoire, comprising :
(a) providing an array of members of the first repertoire juxtaposed with members of the second repertoire which permits interaction of said first repertoire members with said second repertoire members, said array comprising a solid surface, said first repertoire present on a solid surface in a first series of continuous, non-intersecting lines such that each line of said series comprises a member of said first repertoire, and said second repertoire present on a solid surface in a second series of continuous, non-intersecting lines such that each line of said series comprises a member of said second repertoire, such that members of the first repertoire are juxtaposed to members of the second repertoire; and
(b) detecting the lack of an interaction between members of the first and second repertoires, thereby identifying members of the first repertoire that do not interact with members of the second repertoire.

30. A method for screening a first repertoire of members against a second repertoire of members to identify members of the first and second repertoires whose interactions with one another are dependent on the presence or absence of a one or more third molecules, comprising:
(a) providing an array of members of the first repertoire juxtaposed with members of the second repertoire which permits interaction of said first repertoire members with said second repertoire members, said array comprising a solid surface, wherein said first repertoire is present on a solid surface in a first series of continuous, non-intersecting lines such that each line of said series comprises a member of said first repertoire, and said second repertoire is present on a solid surface in a second series of continuous, non-intersecting lines such that each line of said series comprises a member of said second repertoire, such that members of the first repertoire are juxtaposed to members of the second repertoire; and
(b) contacting said first and second repertoires with said one or more third molecules;
(c) detecting interactions between members of the first repertoire and members of the second repertoire in the presence of the one or more third molecules, such that members of the first and second repertoires whose interactions with one another are dependent on the presence or absence of the one or more third molecules are identified.

31. The method of claim 30, wherein said first and second repertoires together are contacted with different concentrations of said one or more third molecules.

32. The method according to claim 30, wherein the interaction of the one or more third molecules with one or more members of the first repertoire permits such members of the first repertoire to interact with one or more members of the second repertoire.

33. The method according to claim 30, wherein the interactions between the members of the first and second repertoire require the simultaneous binding of these members to the one or more molecules.

34. The method according to claim 30 wherein the interactions between the members of the first and second repertoire are enhanced by the presence of the one or more third molecules.

35. The method according to claim 30, wherein the interactions between the members of the first and second repertoire are blocked by the presence of the one or more third molecules.

36. The method according to claim 30, wherein the first and second repertoires are dispensed by a plurality of dispensing events to form the array, and wherein fewer dispensing events are required than the number of interactions to be screened.

37. The method according to claim 36 wherein members of both the first repertoire and the second repertoire are dispensed by a plurality of dispensing events into two or more series of continuous, non-intersecting lines, respectively, each line comprising a member of the first or second repertoires such that the series of lines corresponding to the first repertoire and the series of lines corresponding to the second repertoire intersect such that the number of intersections is greater than the number of dispensing events.

38. A method for determining conditions for a biological interaction, which method comprises creating two or more different sets of variable parameters at the intersections of two or more different sets of intersecting lines, and assaying one or more intersections for a biological interaction, thereby determining the conditions for the biological interaction.

39. A method according to claim 38, wherein the variable parameters are selected from the group consisting of: a buffer composition, a substrate concentration, pH, temperature, the presence of denaturants and the presence of renaturants.

40. A method for screening a first and a second repertoire of enzymes to identify members of the first repertoire and members of the second repertoire which together participate in a two or more step enzymatic reaction that generates a product from a substrate, which method comprises:
(a) providing an array of members of the first repertoire juxtaposed with members of the second repertoire which permits interaction of said first repertoire members and said second repertoire members, said array comprising a solid surface, said first repertoire present on a solid surface in a first series of continuous, non-intersecting lines such that each line of said series comprises a member of said first repertoire, and said second repertoire present on a solid surface in a second series of continuous, non-intersecting lines such that each line of said series comprises a member of said second repertoire, such that members of the first repertoire are juxtaposed to members of the second repertoire;
(b) contacting said array with said substrate; and
(c) detecting the formation of the product at the intersections of the members of the first and second repertoires, thereby identifying members of the first and second repertoires which together participate in a two or more step enzymatic reaction that creates the product from the substrate.

41. A method for screening a first repertoire of cellular populations against a second repertoire of viral populations to identify viral populations among the repertoire of viral populations that infect cellular populations among the repertoire of cellular populations, which method comprises:
(a) providing an array of members of the first repertoire juxtaposed with members of the second repertoire which permits interaction of said first repertoire members with said second repertoire members, said array comprising a solid surface, wherein said repertoire of cellular populations is present on a solid surface in a first series of continuous, non-intersecting lines such that each line of said series comprises a member of said repertoire of cellular populations, and said repertoire of viral populations is present on a solid surface in a second series of continuous, non-intersecting lines such that each line of said series comprises a member of said repertoire of viral populations, such that each of said first series of lines intersects with each of said second series of lines, such that members of the repertoire of cellular populations are juxtaposed to members of the repertoire of viral populations which permits interaction of said repertoire of cellular populations and said repertoire of said viral populations; and
(b) detecting viral infection in the plurality of cellular populations, thereby identifying viral populations among the repertoire of viral populations that infect cellular populations among the repertoire of cellular populations.

42. A method for screening a plurality of different cellular fractions against one another to identify cellular fractions of said plurality that contain components which interact with components in other cellular fractions of said plurality, which method comprises:
(a) providing an array, comprising a solid surface, comprising a first repertoire of cellular fractions and a second repertoire of cellular fractions wherein said first repertoire is present on a solid surface in a first series of continuous, non-intersecting lines such that each line of said series comprises an individual cell fraction of said first repertoire, and said second repertoire is present on a solid surface in a second series of continuous, non-intersecting lines such that each line of said series comprises an individual cell fraction of said second repertoire, such that each of said first series of lines intersects with each of said second series of lines, such that cell fractions of the first repertoire are juxtaposed to cell fractions of the second repertoire which permits interaction of said first and second repertoire cellular fractions; and
(b) detecting the interaction of different cellular fractions at sites where the different cellular fractions are juxtaposed, thereby identifying cellular fractions of said plurality that contain components which interact with components in other cellular fractions of said plurality.

43. A method for screening a plurality of different cellular populations against one another to identify cellular populations of said plurality that interact with other cellular populations of said plurality, which method comprises:
(a) providing an array, comprising a solid surface, comprising a first repertoire of cellular populations and a second repertoire of cellular populations wherein said first repertoire is present on a solid surface in a first series of continuous, non-intersecting lines such that each line of said series comprises an individual cell population of said first repertoire, and said second repertoire is present on a solid surface in a second series of continuous, non-intersecting lines such that each line of said series comprises an individual cell population of said second repertoire, such that each of said first series of lines intersects with each of said second series of lines, such that cell populations of the first repertoire are juxtaposed to cell populations of the second repertoire which permits interaction of said first and second repertoire cellular populations; and
(b) detecting the interaction of different cellular populations at sites where the different cellular populations are juxtaposed, thereby identifying cellular populations of said plurality that interact with other cellular populations of said plurality.

44. A method for screening each member of a polypeptide repertoire against each other member of said polypeptide repertoire, in order to identify members of the polypeptide repertoire that interact with other members of the polypeptide repertoire, which method comprises:
(a) providing an array, comprising a solid surface, comprising a first repertoire of polypeptides and a second repertoire of the same polypeptides wherein said first repertoire is present on a solid surface in a first series of continuous, non-intersecting lines such that each line of said series comprises a member of said first repertoire, and said second repertoire is present on a solid surface in a second series of continuous, non-intersecting lines such that each line of said series comprises a member of said second repertoire, such that each of said first series of lines intersects with each of said second series of lines, such that polypeptides of the first repertoire are juxtaposed to polypeptides of the second repertoire which permits interaction of said first and second repertoire polypeptides; and
(b) detecting the interaction of different members of the polypeptide repertoire at sites where the different members are juxtaposed, thereby identifying members of the polypeptide repertoire that interact with other members of the polypeptide repertoire.

45. The method according to claim 1 or 44, which method uses a yeast two-hybrid system to identify those members of the repertoires of molecules that interact with one another.

46. A method for creating a combinatorial library comprising members of a first repertoire of polypeptides paired with members of a second repertoire of polypeptides, which method comprises:
(a) providing an array comprising a solid surface wherein a plurality of host cells comprising a plurality of nucleotide sequences encoding a first repertoire of polypeptide members is present on a solid surface in a first series of continuous, non-intersecting lines such that each line of said series comprises a member of said first repertoire of polypeptide members, and a plurality of nucleotide sequences encoding a second repertoire of polypeptide members is present on a solid surface in a second series of continuous, non-intersecting lines such that each line of said series comprises a member of said second repertoire of polypeptide sequences of to create an array, such that each of said first series of lines intersects with each of said second series; and
(b) transforming the cells containing the nucleotide members of the first repertoire with the nucleotide sequences that encode the members of the second repertoire where the two repertoires intersect; and
(c) expressing the nucleotide sequences to produce the corresponding polypeptides of the first and second repertoires; thereby creating a combinatorial library consisting of members of the first repertoire of polypeptides paired with members of the second repertoire of polypeptides.

47. A method for screening the combinatorial library for members of a first repertoire of polypeptides that interact with members of a second repertoire of polypeptides, wherein said combinatorial library is generated by a method comprising providing an array comprising a solid surface and a plurality of host cells comprising a plurality of nucleotide sequences encoding the first repertoire of polypeptide members present on a solid surface in a first series of continuous, non-intersecting lines such that each line of said series comprises a member of said first repertoire of polypeptide members, and a plurality of nucleotide sequences encoding the second repertoire of polypeptide members present on a solid surface in a second series of continuous, non-intersecting lines such that each line of said series comprises a member of said second repertoire of polypeptide sequences of to create an array, such that each of said first series of lines intersects with each of said second series; transforming the cells containing the nucleotide members of the first repertoire with the nucleotide sequences that encode the members of the second repertoire where the two repertoires intersect; and expressing the nucleotide sequences to produce the corresponding polypeptides of the first and second repertoires; thereby creating a combinatorial library consisting of members of the first repertoire of polypeptides paired with members of the second repertoire of polypeptides, the method comprising the step of detecting an interaction between the polypeptide members of the first and second repertoires, thereby identifying members of the first repertoire that interact with members of the second repertoire.

48. A method for creating a combinatorial library consisting of members of a first repertoire of polypeptides paired with members of a second repertoire of polypeptides, which method comprises:
(a) providing an array comprising a solid surface and a plurality of host cells comprising a plurality of nucleotide sequences encoding a first repertoire of polypeptide members present on a solid surface in a first series of continuous, non-intersecting lines such that each line of said series comprises a member of said first repertoire of polypeptide members, and a plurality of viruses containing a plurality of nucleotide sequences encoding a second repertoire of polypeptide members present on a solid surface in a second series of continuous, non-intersecting lines such that each line of said series comprises a member of said second repertoire of polypeptide sequences of to create an array, such that each of said first series of lines intersects with each of said second series;
(b) infecting the cells containing the nucleotide members encoding the first repertoire with the viruses that contain the nucleotide members encoding the second repertoire where the first and second series of lines intersect; and
(c) expressing the nucleotide sequences to produce the corresponding polypeptides of the first and second repertoires, thereby creating a combinatorial library consisting of members of the first repertoire of polypeptides paired with members of the second repertoire of polypeptides.

49. A method of screening the combinatorial library to identify members of a first repertoire of polypeptides that interact with members of a second repertoire of polypeptides, wherein said combinatorial library is generated by the method comprising providing an array comprising a solid surface and a plurality of host cells comprising a plurality of nucleotide sequences encoding the first repertoire of polypeptide members present on a solid surface in a first series of continuous, non-intersecting lines such that each line of said series comprises a member of said first repertoire of polypeptide members, and a plurality of viruses containing a plurality of nucleotide sequences encoding the second repertoire of polypeptide members present on a solid surface in a second series of continuous, non-intersecting lines such that each line of said series comprises a member of said second repertoire of polypeptide sequences of to create an array, such that each of said first series of lines intersects with each of said second series; infecting the cells containing the nucleotide members encoding the first repertoire with the viruses that contain the nucleotide members encoding the second repertoire where the first and second series of lines intersect; and expressing the nucleotide sequences to produce the corresponding polypeptides of the first and second repertoires, thereby creating a combinatorial library consisting of members of the first repertoire of polypeptides paired with members of the second repertoire of polypeptides, the method comprising the step of detecting an interaction between polypeptide members of the first and second repertoires, whereby members of the first repertoire that interact with members of the second repertoire are identified.

50. A method for creating a yeast two hybrid library consisting of members of a first repertoire of polypeptides paired with members of a second repertoire of polypeptides, which method comprises:
(a) providing an array comprising a solid surface and a first plurality of yeast cells comprising a plurality of nucleotide sequences encoding a first repertoire of polypeptide members present on a solid surface in a first series of continuous, non-intersecting lines such that each line of said series comprises a member of said first repertoire of polypeptide members, and a second plurality of yeast cells containing a plurality of nucleotide sequences encoding a second repertoire of polypeptide members present on a solid surface in a second series of continuous, non-intersecting lines such that each line of said series comprises a member of said second repertoire of polypeptide sequences of to create an array, such that each of said first series of lines intersects with each of said second series;
(b) allowing the yeast cells containing the members of the first repertoire to mate with the yeast cells containing the members of the second repertoire where the two repertoires intersect; and
(c) expressing the nucleotide sequences to produce the corresponding polypeptides of the first and second repertoires, thereby creating a yeast two hybrid library comprising members of a first repertoire of polypeptides paired with members of a second repertoire of polypeptides.

51. A method of screening a combinatorial library to identify members of a first repertoire of polypeptides that interact with members of a second repertoire of polypeptides, wherein said combinatorial library is generated by the method comprising providing an array comprising a solid surface and a first plurality of yeast cells comprising a plurality of nucleotide sequences encoding the first repertoire of polypeptide members present on a solid surface in a first series of continuous, non-intersecting lines such that each line of said series comprises a member of said first repertoire of polypeptide members, and a second plurality of yeast cells containing a plurality of nucleotide sequences encoding the second repertoire of polypeptide members present on a solid surface in a second series of continuous, non-intersecting lines such that each line of said series comprises a member of said second repertoire of polypeptide sequences of to create an array, such that each of said first series of lines intersects with each of said second series; allowing the yeast cells containing the members of the first repertoire to mate with the yeast cells containing the members of the second repertoire where the two repertoires intersect; and expressing the nucleotide sequences to produce the corresponding polypeptides of the first and second repertoires, thereby creating a yeast two hybrid library comprising members of the first repertoire of polypeptides paired with members of the second repertoire of polypeptides, the method comprising the step of detecting an interaction between the polypeptide members of the first and second repertoires, whereby members of the first repertoire that interact with members of the second repertoire are identified.

52. A method of creating a combinatorial chemical library comprising:
(a) providing an array comprising a solid surface and a first repertoire of chemical groups comprising a first reactive group present on said solid surface in a first series of continuous, non-intersecting lines;
(b) reacting said solid surface with a reagent to modify said first reactive group to render said first reactive group capable of forming a chemical bond with a second reactive group;
(c) depositing a second repertoire on said solid surface comprising a second reactive group capable of forming a chemical bond with said first reactive group, wherein said second repertoire is deposited in a second series of continuous, non-intersecting lines, such that each line of said first series intersects with each line of said second series, such that each member of said first repertoire is juxtaposed to each member of said second repertoire, wherein a reactive group of said second repertoire forms a chemical bond with a reactive group of said second repertoire thereby producing a combinatorial chemical library.

53. A method for screening a first repertoire comprising a combinatorial chemical library against a repertoire of members to identify members of the first repertoire which interact with members of the second repertoire, wherein said combinatorial chemical library is produced by a method comprising providing an array comprising a solid surface and a first repertoire of chemical groups comprising a first reactive group present on said solid surface in a first series of continuous, non-intersecting lines; reacting said solid surface with a reagent to modify said first reactive group to render said first reactive group capable of forming a chemical bond with a second reactive group; depositing a second repertoire on said solid surface comprising a second reactive group capable of forming a chemical bond with said first reactive group, wherein said second repertoire is deposited in a second series of continuous, non-intersecting lines, such that each line of said first series intersects with each line of said second series, such that each member of said first repertoire is juxtaposed to each member of said second repertoire, wherein a reactive group of said second repertoire forms a chemical bond with a reactive group of said second repertoire thereby producing a combinatorial chemical library, the method comprising the step of juxtaposing said combinatorial chemical library to said second repertoire and detecting an interaction between members of said combinatorial library and members of said second repertoire, thereby identifying members of said first repertoire comprising said combinatorial library which interact with members of said second repertoire.
